(19) **Europäisches Patentamt**
**European Patent Office**
**Office européen des brevets**

(11) **EP 2 161 278 B1**

(12) **EUROPEAN PATENT SPECIFICATION**

(45) Date of publication and mention
of the grant of the patent:
**23.05.2012 Bulletin 2012/21**

(51) Int Cl.:
**C07K 14/00** (2006.01)

(21) Application number: **08172017.9**

(22) Date of filing: **17.12.2008**

(54) **Single-chain coiled coil scaffold**

Gewundenes Spulengerüst aus einer einzelnen Kette

Échafaudage à bobine à une seule chaîne

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **08.09.2008 EP 08163895**

(43) Date of publication of application:
**10.03.2010 Bulletin 2010/10**

(73) Proprietor: **Complix N.V.
3590 Diepenbeek (BE)**

(72) Inventors:
• **Desmet, Johan
B-8500 Kortrijk (BE)**
• **Lasters, Ignace
B-2018 Antwerpen (BE)**

(74) Representative: **Taormino, Joseph Paul
Hoffmann - Eitle
Patent- und Rechtsanwälte
Arabellastrasse 4
81925 München (DE)**

(56) References cited:
**WO-A-99/16883          WO-A-2005/077103
WO-A-2005/118886    WO-A-2009/030780**

• **EFIMOV VP ET AL: "Fibritin Encoded by
Bacteriophage T4 Gene wac has a Parallel Triple-
stranded .alpha.-Helical Coiled-coil Structure"
JOURNAL OF MOLECULAR BIOLOGY, vol. 242,
1994, pages 470-486, XP002523719**
• **BOSCH B J ET AL: "Severe acute respiratory
syndrome coronavirus (SARS-CoV) infection
inhibition using spike protein heptad repeat-
derived peptides" PROCEEDINGS OF THE
NATIONAL ACADEMY OF SCIENCES OF USA,
NATIONAL ACADEMY OF SCIENCE,
WASHINGTON, DC.; US, vol. 101, no. 22, 1 June
2004 (2004-06-01), pages 8455-8460,
XP002994902 ISSN: 0027-8424**
• **KIYOKAWA T ET AL: "Engineering of the
Hydrophobic Core of an .alpha.-Helical Coiled
Coil" BIOPOLYMERS, vol. 55, no. 5, 2000, pages
407-414, XP008105332**
• **HARRIS NL ET AL: "Four Helix Bundle Diversity
in Globular Proteins", JOURNAL OF
MOLECULAR BIOLOGY, vol. 236, 1994, pages
1356-1368,**

**Description**

**Field of the invention**

The present invention is in the field of molecular biology and is related to a thermodynamically stable, single-chain protein (aceous) scaffold that consists of a three-stranded, parallel, alpha-helical coiled coil structure. This scaffold, presenting possible therapeutic, diagnostic or purification purposes, is used in the field of drug discovery, analytical research, purification technology and as a model for improving research and development of new proteinaceous (protein-like) scaffold structures.

**Background of the invention**

Proteinaceous (protein-like) molecular compounds (molecules) are able to bind proteinaceous target compounds (target molecules, targets) such as flexible peptides in solution, peptides immobilized onto a solid support, linear peptide fragments in a protein, or folded proteins. These molecular compounds have a broad spectrum of possible applications. For example, they are suited to be used as therapeutic compounds (e.g., inhibitors), detection probes (e.g., detection of a recombinant protein) and purification probes (e.g., in affinity chromatography). In view of the continuous evolution in these areas, there is an ongoing need for new compounds that can bind specific target molecules.

Current therapeutic treatments are generally based on pharmacological or biotechnological compounds, the latter including either immunoglobulin(-derived) or non-immunoglobulin compounds. The production, purification, testing and optimization of both types of biotechnological compounds is generally labor-intensive, riskful and expensive. Accordingly, there is a need for new biotechnological compounds with specific biological activity, as well as improved methods for the production, purification, testing and optimization of such compounds.

EFIMOV VP ET AL. ( "Fibritin Encoded by Bacteriophage T4 Gene wac has a Parallel Triple-stranded .alpha.-Helical Coiled-coil Structure" JOURNAL OF MOLECULAR BIOLOGY, vol. 242, 1994, pages 470-486) disclose the amino acid sequence of the single-chain protein fibritin that has a parallel triple-stranded coiled coil structure, wherein heptad substructures are separated by short linker regions that have a high proportion of glycine and proline residues.

**Aims of the invention**

The present invention aims to provide a new class of compounds as claimed that does not present the drawbacks of the state of the art especially a class of compound that does not present concentration dependence and that is easily producible (synthesized), preferably via recombinant methods (i.e., using molecular biological techniques), especially synthesis of trimeric coiled coil scaffolds in peptidic form.

**Summary of the invention**

The present invention is related to proteinaceous molecular compounds consisting of a triple-stranded, parallel, alpha-helical coiled coil scaffold structure in solution. Such molecules can be very stable and tolerant to amino acid substitutions. Accordingly, they meet the basic requirements of a protein-based scaffold. Protein-based scaffold molecules are often considered as the 'next-generation' class of compounds for molecular recognition, which increasingly compete with immunoglobulin-based compounds. Accordingly, these compounds offer an alternative approach to immunoglobulins, and an additional type of protein-based (proteinaceous) scaffold.

Coiled coil structures have found various applications in the medical and biotechnological fields (e.g., as gene regulators, antibody stabilizers, anticancer drugs, purification tags, hydrogels). The usage of two-stranded coiled coil scaffolds has been proposed for the construction of combinatorial libraries and for vaccine design. However, the present invention does not relate to two-stranded coiled coils. Instead, the compounds of the present invention form triple-stranded coiled coils. The triple-stranded alpha-helical coiled coil scaffold of the invention further shows a unique feature: it possesses a highly designable groove in between each pair of interacting alpha-helices.

The present invention discloses that the latter feature makes this type of scaffold well-suited for binding to peptidic target compounds. Since the development of peptide-binding molecules is generally known as a difficult problem, the present invention discloses a valuable, innovative and non-obvious alternative to existing approaches.

Triple-stranded alpha-helical coiled coil complexes are formed in solution by the association of individual (separate, monomeric) peptide molecules into trimers. The individual peptides typically comprise one or more heptad repeats (heptad units, heptads) which provide the thermodynamic driving force for such association.

However, an important practical problem encountered with the formation of trimeric complexes is the fact that such reactions are extremely dependent on the concentration. Therefore, unless the thermodynamic driving force is extremely strong (i.e., only if the heptad form extremely tight interactions), one has to apply high concentrations in order for the trimeric complex to form. High concentrations can have multiple adverse (unwanted) effects when applied (administered) as pharmaceutical compounds.

Since the amino acid sequence of single-chain proteins can be specified (chosen) at the level of individual amino acids, it becomes possible to create scaffolds comprising a triple-stranded coiled coil structure wherein each of the constituting coiled coil strands have a different amino acid sequence. In contrast, peptidic coiled coils being formed by association of peptides with different amino acid sequences is extremely hard to control.

Therefore, the present invention provides a practical solution to the problem of creating heterotrimeric coiled coil structures.

Non-covalent interactions of proteins with other molecules are at the basis of all biological processes. These interactions determine the strength and specificity of molecular recognition events. Accordingly, rational application of the principles behind such interactions, in combination with biotechnological methods, may provide a means to interfere (intervene) with biological processes, or enable detection or purification of compounds of interest.

The present invention relates to stable (stably folded, stably associated) scaffold molecules, the stability of which is mediated through specific non-covalent intramolecular interactions. Other embodiments of the present invention relate to scaffold molecules which associate with (bind to) target molecules of high pharmaceutical or diagnostic interest, this association likewise being mediated through specific non-covalent interactions.

The inventors have obtained the compound of the invention that presents the minimal requirements for such scaffolds, a stable (thermodynamically stable, thermally stable, chemically stable, pH-insensitive) three-dimensional fold (3-D fold, 3-D structure).

However, particular amino acids (amino acid residues, residues) can be substituted (mutated, changed, varied) without compromising the essential properties of this scaffold, i.e., a high stability and preservation of its three-dimensional structure. These scaffold molecules can be mutated so as to allow them to bind (bind to, bind with) specific target molecules.

The present invention relates to scaffold molecules (molecular compounds) that are able to interfere with (influence, modify) biological processes through impeding (blocking, inhibiting) natural chemical reactions or natural molecular recognition events, or through creation of non-natural molecular recognition events.

The single-chain protein scaffold according to the present invention consists of a full amino acid sequence of the formula HRS1-L1-HRS2-L2-HRS3, wherein HRS1, L1, HRS2, L2 and HRS3 represent amino acid sequence fragments that are covalently and consecutively interconnected in the order of the said formula, wherein

a) these fragments HRS1, HRS2 and HRS3 are heptad repeat sequences, and
b) the two fragments L1 and L2 are structurally flexible linker sequences which have an amino acid composition comprising at least 70 % of the amino acids selected from the group glycine, serine, threonine, alanine, proline, histidine, asparagine, aspartic acid, glutamine, glutamic acid, lysine, arginine, and
c) said full amino acid sequence spontaneously and stably folds in solution by way of the HRS1, HRS2 and HRS3 fragments that constitute a triple-stranded, parallel, alpha-helical coiled coil structure, and
d) each of the linkers L1 and L2 bridges between two distal ends of said coiled coil structure, and
e) the amino acid residues near the termini of each of the linkers L1 and L2 promote formation of a turn in between the connected alpha-helix and the remainder of the linker, and
f) turn-forming amino acids are selected from the group consisting of proline, glycine, asparagine and aspartic acid, and
g) such turn is made at either end of said linkers.

Another aspect of the present disclosure relates to a method to generate or to optimize such compounds, to detect molecular compounds of interest in a study sample and to isolate molecular compounds of interest from a study sample.

Instances of biological interference include, without limitation, blocking of human receptors, binding to pathogenic species, and binding to disease- or disorder-related proteins. Such type of biological interference is typically intended to curate severe diseases or disorders. These applications belong to the field of therapeutic research and development.

Specific probe molecules (probes) can be applied to detect a presence of an analyte of interest (target analyte) in a given sample of interest (study sample), include, without limitation, experimental analyses of samples of human, animal, plant, bacterial, viral, biotechnological or synthetic origin. Such samples typically contain biomolecules (e.g., polypeptides, polynucleotides, polysaccharides, hormones, vitamins or lipids, or derivatives thereof) that can interact specifically with a selected probe molecule. The latter interaction typically gives rise to a characteristic (e.g., spectroscopic or radioactive) signal, indicative of the presence of said target analyte in said study sample. These applications belong to the field of analytical research and development.

The number of combinations of different types of probes and targets that are effectively used in medical and biotechnological applications is (virtually) unlimited. In view of the continuous evolution in these areas, there is an ongoing need for new analytical tools (e.g., probes) with desired physico-chemical properties (e.g., specificity, affinity, stability, solubility), as well as improved methods for the production, purification, testing and optimization of such compounds.

Instances wherein specific ligand molecules (ligands) are applied to retain (extract, isolate, purify, filter) other molecules of interest (targets, target analytes) in a given sample of interest (crude sample) include, without limitation, samples of human, animal, plant, bacterial, viral, biotechnological or synthetic origin containing biomolecules (e.g., polypeptides, polynucleotides, polysaccharides, hormones, vitamins or lipids, or derivatives thereof) that can interact (associate) with high specificity with selected ligand molecules, where the latter are separated, or can be separated, from the crude sample (e.g., by attachment onto a solid support or by precipitation), for the purpose of co-separating the target molecules from the crude sample. These applications belong to the field of purification technology.

More specific examples of purification methods include affinity chromatography and immunoprecipitation. In view of the continuous evolution in these areas, there is an ongoing need for new ligands for purification with desired physico-chemical properties (e.g., specificity, affinity, stability, solubility), as well as improved methods for the production, purification, testing and optimization of such compounds.

Immunoglobulin molecules (antibodies, including homologs and derivatives) are widely used in all of the aforementioned fields. They can recognize a diverse repertoire of target antigens and bind with great specificity. However, they suffer from many disadvantages including

(i) the requirement of laboratory animals for the production of polyclonal antibodies (immunization technology),
(ii) the requirement of complicated methods to derive monoclonal antibodies from polyclonal ensembles (hybridoma technology),
(iii) the non-human nature of antibodies obtained through immunization of animal vertebrates (causing potential problems related to immunogenicity),
(iv) difficulties to convert non-human antibodies into human or humanized variants (e.g., causing affinity loss),
(v) alternative production methods based on protein library display and selection usually do not yield high-affinity products, thereby requiring additional affinity enhancement steps,
(vi) all production methods are time-consuming and require highly specialized researchers,
(vii) standard immunoglobulins may experience steric difficulties to reach their target binding sites *in vivo,* as opposed to *in vitro* test systems,
(viii) native and, to a lesser extent, engineered antibodies may have suboptimal properties relating to hydrophobicity, immunogenicity, bivalency or effector function,
(ix) therapeutic antibodies must be stored at near freezing temperatures,
(x) immunoglobulin products are generally digested in the gut and must therefore be administered via injection or infusion,
(xi) antibodies experience difficulties to permeate the blood-brain barrier.

Accordingly, there is a need for alternative, non-immunoglobulin compounds that have similar qualities but fewer weaknesses. The present invention contemplates the use of a specific type of protein (proteinaceous) scaffold and corresponding specific molecular compounds with binding specificities comparable to those of immunoglobulins, but with a totally distinct composition and structure, and lacking some of the immunoglobulins' weaknesses.

The term 'scaffold' is used within the context of the present invention to denote 'a specific, conformationally (structurally) and thermodynamically (thermally and chemically) stable proteinaceous (protein-like or protein) molecule with a specific, fixed (invariable, invariant) three-dimensional (3-D, tertiary) structure (spatial arrangement of constituting elements) consisting of one or more protein or proteinaceous polypeptide chains, this structure being demonstrably tolerant to a variety of single and multiple amino acid substitutions at a variety of amino acid residue positions', where 'tolerant' is to be understood in the sense that the integrity (correctness) of the structure remains essentially unaltered upon performing said amino acid substitutions.

Non-immunoglobulin protein-based (proteinaceous) scaffold molecules are considered in the field as a 'next-generation' class of compounds for molecular recognition. They are mostly derived from natural protein molecules which have been selected on basis of preferred physico-chemical properties and available experimental data. Examples of this class of compounds are listed in [Hosse et al. Protein Sci 2006, 15:14-27].

Because of the high stability of protein-based scaffold structures, large libraries (scaffold-based libraries, scaffold libraries) of molecules with essentially the same tertiary structures and slightly different sequences can be constructed. Alternatively, surface residues can be varied by rational or semi-rational protein engineering methods. Appropriate selection methods can be applied for the purpose of identifying variants (scaffold derivatives, specific molecular compounds) with highly desired binding properties (e.g., affinities and specificities) similar to immunoglobulins. Protein-based scaffold molecules have been ascribed numerous advantages over immunoglobulins including, for example, their relatively small size, high structural stability and absence of post-translational modifications. These features considerably facilitate their synthesis, purification and storage. Moreover, high-affinity compounds can be generated without the need to proceed via an immunization step.

Certain disadvantages of protein-based scaffold structures have been reported as well, notably their premature stage of development and lack of broad validation. Further, because of their low molecular weight, they are generally cleared rapidly from the bloodstream. It can also be argued that scaffold-based proteins, especially the ones derived from non-human proteins, pose a risk.to elicit strong immunologic responses. Next, their small sizes put certain limits to their 'engineerability': often only isolated surface loops are amenable to variation, which significantly restricts the number of displayed (displayable) 3-D patterns. Of particular relevance to the present invention is the fact that very few scaffold-based molecules are able to bind peptide ligands [Hosse et al. *ibid*]. Accordingly, there is a need for novel protein-based scaffold molecules with enhanced properties. There is a particular need for such type of molecules which can recognize small peptides. One aspect of the present invention relates to a particular type of protein-based scaffold that is largely insensitive to substitution of surface residues and standard protein engineering actions. Another aspect relates to scaffold-derived compounds, including compounds that tightly bind peptides comprising a sequence pattern of interest. Yet another aspect relates to scaffold-derived compounds, including compounds that tightly bind proteins comprising a surface pattern of interest. All embodiments of the present invention relate to a specific type of protein structure (3-D structure, tertiary structure, fold) that has so far not been exploited as a highly mutatable scaffold.

Protein-based scaffold molecules ideally consist of a stable framework structure (scaffold framework) and one or more regions that can be varied by amino acid substitution (variable regions) without compromising folding of the molecule, i.e., without preventing correct formation of its framework.

As will be acknowledged by persons skilled in the field of protein physics, most natural proteins or protein domains do not have an exceptionally stable tertiary structure. Natural proteins or domains mostly unfold upon heating, with transition temperatures well below 100 °C. They are also generally susceptible to protease digestion. Furthermore, they are generally not tolerant to a broad range of amino acid substitutions, in the sense that inconsiderate or random introduction of one or more substitutions may significantly destabilize or even disrupt the framework. It is therefore in general not obvious to directly utilize natural proteins or domains as scaffold molecules. The present invention relates to a scaffold which is remotely based (inspired) on a structural motif (structural framework) that exists in nature (i.e., the trimeric coiled coil), but which has been thoroughly engineered in order for it to meet the basic requirement of a useful scaffold, namely, a high stability and robustness of its framework.

The alpha-helical coiled coil forms a special type of 3-D structural framework (structural motif, fold). The coiled coil fold occurs in a wide variety of proteins including motor proteins, DNA-binding proteins, extracellular proteins and viral fusion proteins [Burkhard et al. Trends Cell Biol 2001, 11:82-88]. It has been estimated that 3 to 5 % of all amino acids in natural proteins are part of a coiled coil structure [Wolf et al. Protein Sci 1997, 6:1179-1189]. Coiled coils have been functionally characterized as folding (assembly, oligomerization) motifs, i.e., formation of a coiled coil structure drive in many instances the non-covalent association of different protein chains. Coiled coils have been structurally characterized as 2-, 3-, 4- or 5-stranded assemblies of alpha-helices arranged in parallel, antiparallel or mixed topologies [Lupas Trends Biochem Sci 1996, 21:375-382]. The helices are slightly wrapped (coiled) around each other in a left-handed manner, termed supercoiling.

Alpha-helical coiled coils have been further characterized at the level of their amino acid sequences in that each helix is constituted of a series of heptad repeats. A heptad repeat (heptad unit, heptad) is a 7-residue sequence motif which can be encoded as hpphppp, and wherein each h represents a (potentially different) hydrophobic residue (mostly Leu, Ile or Val) and each p is a (potentially different) polar residue (e.g., Ser, Thr, Asn, Gln, Asp, Glu, His, Arg or Lys). Occasionally (infrequently), p-residues are observed at H-positions, and vice versa.

Coiled coils have been thermodynamically characterized as follows. When the sequence folds into an alpha-helix, the hydrophobic residues (H) form a hydrophobic seam, whereas the polar residues (p) form a polar face. The hydrophobic seams of different alpha-helices, when associated into a coiled coil, form a central hydrophobic core (center, interior, inner part). Formation of this core, in combination with orientation of the polar faces toward solvent, is assumed to provide the main thermodynamic driving force required for stable association, although certain non-core residues may enhance stability as well.

The present invention also relates to polypeptide fragments (amino acid sequence fragments, amino acid sequences) of more than 50, 60, 70, 80, 90, 100, 110, 120 or 130 amino acids presenting some binding properties as the full length sequence of the single action compound of the invention amino acids comprising multiple heptad repeat motifs. These embodiments further relate to triple-stranded (3-stranded, trimeric), parallel, alpha-helical coiled coil structures. Preferably, stable association of the polypeptide chains in 3-stranded parallel alpha-helical coiled coil structures and coiled coils for which the core residues provide the main driving force for stable association.

Alpha-helical coiled coils have found widespread applications in the medical and biotechnological fields. They have been exploited to function as temperature-sensing gene regulators [Naik et al. Biosens Bioelectron 2001, 16: 1051-1057], antibody stabilizers [Arndt et al. J Mol Biol 2001, 312:221-227], anticancer drugs [Moitra et al. Genes Dev 1998, 12:3168-3181], purification tags [Müller et al. Methods Enzymol 2000, 328:261-282], hydrogels [Wang et al. Nature 1999, 397:417-420] and linker systems [Ryadnov et al. J Am Chem Soc 2003, 125:9388-9394] [reviewed in Mason and Arndt ChemBioChem 2004, 5:170-176].

Despite the facts that

(i) the technological versatility of helical coiled coils has been acknowledged, and proven, in a variety of different applications, and
(ii) the concept of drug design based on scaffolds other than helical coiled coils has been proven to be successful in a number of cases, few, if any, examples of successful alpha-helical coiled coil scaffold-based drugs have been reported so far in the literature.

US Patent No 5,824,483 has claimed the usage of a combinatorial library of conformationally restricted peptides, wherein the said peptides associate (fold) into an alpha-helical coiled coil dimer. Peptide sequences of the scaffold are designed such that they simultaneously fulfill a double role:

(i) a subset of the composing amino acid residues, denoted invariant residues, is selected for (serves to) stabilize the dimeric coiled coil, this subset including both amino acid residues suitable to form the hydrophobic core (center, interior) of the coiled coil dimer, and residues that form a covalent intrachain bond (i.e., a covalent bond between residues in one of the constituting peptides, effective to stabilize that peptide in its alpha-helical conformation), and
(ii) a subset of the composing amino acid residues, denoted variant residues, is selected among the solvent-exposed residues, and is varied (substituted, mutated) so as to create a unique variation of amino acid residues in the exposed region of at least one of the constituting peptides, this variation involving at least 1000 library members. Thus, the inventors essentially claimed a combinatorial library that is based on a dimeric (2-stranded) alpha-helical coiled coil scaffold. In contrast, all embodiments of the present invention relate to the usage of a 3-stranded (triple-stranded) alpha-helical coiled coil scaffold, and not to coiled coil dimers. Furthermore, none of the embodiments of the present invention, unlike the referenced invention (US Patent No 5,824,483) includes (mentions, requires) the usage of intrachain covalent bonds other than the obvious bonds of a standard polypeptide chain.

Triple-stranded alpha-helical coiled coils are less frequently observed in natural proteins than are dimeric coiled coils [Wolf et al. Protein Science 1997, 6:1179-1189]. Triple-stranded coiled coils are also less frequently used for biotechnical or medical purposes [Mason and Arndt ChemBioChem 2004, 5:170-176]. Of particular relevance with respect to the present invention is the fact that 3-stranded alpha-helical coiled coils so far have not been used as probes (ligands, e.g., agonists, antagonists, inverse agonists, inhibitors, detection probes, purification probes, diagnostic probes, etc.) for binding to selected target molecules.

Instead, their intrinsic scaffold properties, in the sense of 'a stably folded structural unit able to carry other elements', has been exploited almost exclusively under the form of fusion constructs (fusion proteins). More specifically, 3-stranded coiled coil peptides were fused (covalently attached, coupled, linked) to peptides with low trimerization propensity in order to enhance trimerization [Eckert and Kim Proc Natl Acad Sci USA 2001, 98: 11187-11192], or coupled to already trimeric complexes in order to further enhance stability of the complex [Yin et al. Nature 2006, 439:38-44]. The US Patent application 2003/002795 has claimed the (not conclusively demonstrated) vaccinal usage of coiled coil structural scaffolds for the purpose of generating structure-specific peptides which present (display) potentially immunogenic epitopes that are derived (copied, transferred, transplanted, grafted, spliced) from native coiled coil proteins of microbial origin. However, the cited US Patent application application 2003/002795 exclusively considers the usage of dimeric, and not trimeric, coiled coils. Furthermore, this US Patent application does not consider the usage of any type of coiled coil structure in single-chain format (i.e., wherein the constituting alpha-helices are interconnected by flexible linker fragments, see further below). In contrast, all embodiments of the present invention exclusively relate to single-chain coiled coil scaffolds.

Triple-stranded alpha-helical coiled coil scaffold structures exhibiting a high thermal stability have been developed. For example, the Ile-zipper of Suzuki et al. [Protein Eng 1998, 11:1051-1055] was shown to have a melting (unfolding, transition) temperature exceeding 80 °C. Similarly, Harbury et al. [Science 1993, 262:1901-1407; Nature 1994, 371:80-83] designed a GCN4-derived triple-stranded coiled coil, named GCN4-pII, which was found stable in the crystal and in solution. Further, heterotrimeric parallel coiled coils were also designed with success [Nautiyal and Alber Protein Sci 1999, 8:84-90].

The main rules for peptides to fold into trimeric parallel configurations are also grossly known [Yu Adv Drug Deliv Rev 2002, 54:1113-1129]. While this may suggest that the design of 3-stranded parallel coiled coils is relatively straightforward, many studies have reported serious difficulties. For example, a coiled coil that was designed as a parallel dimer was observed in the crystal structure as an antiparallel trimer [Lovejoy et al. Science 1993, 259:1288-1293].

Further, the requirement of a trigger sequence for enhancing the folding kinetics has been a matter of debate [Yu *ibid*]. In addition, the thermal unfolding process does not always follow a simple two-state mechanism [Dragan and Privalov J mol Biol 2002, 321:891-908] and the assembly (folding) process is occasionally very slow [Dragan et al. Biochemistry 2004, 43:14891-14900]. Accordingly, in view of the many unexpected results obtained by skilled researchers, it can be concluded that the design and application of triple-stranded parallel alpha-helical coiled coil molecules is absolutely not obvious. In addition, not any person skilled in the field has proposed or demonstrated that such coiled coil molecules may be useful as scaffold molecules being tolerant to a variety of single and multiple substitutions. In addition, not any person skilled in the field has realized or demonstrated that such coiled coil molecules may be useful as scaffold molecules that can be converted, by way of substitution of the non-core residues, into molecular compounds (ligands) that bind to target molecules of high value in the fields of therapeutics, diagnostics or purification technology.

Further with respect to scaffold molecules, Skerra [J Mol Recognit 2000, 13:167-187] teaches some generally preferred features of protein-based scaffolds: *"According to practical demands they should be based on monomeric and small polypeptides which are robust, easily engineered, and efficiently produced (...)"*.

The present invention relies in part on the advantageous properties of trimeric coiled coil complexes with respect to these generally preferred features of proteinaceous scaffolds. However, peptidic trimeric coiled coil complexes suffer from one very important disadvantage, i.e., the fact that they are not monomeric, but composed of three individual peptides that need to associate in order to form the (functionally active) trimeric structure. Unless this association is extremely tight (thermodynamically favorable), or unless one applies high concentrations (which is often not desirable because of the high risks of side-effects and high costs), such trimeric scaffold is of relatively limited use for diagnostic and therapeutic applications.

Despite their limitations, trimeric coiled coil scaffolds in peptidic format (peptidic trimeric coiled coils) can be extremely valuable to generate information relating to the robustness of this type of fold (scaffold) in terms of tolerance to changes in pH, temperature, ionic strength and amino acid substitution. The latter is evidenced by the Examples.

Usage of peptidic triple-stranded coiled coil scaffolds is proposed for scientific purposes as well as advanced medical and diagnostic applications, while at the same time attempting to find a practical solution to the inherent disadvantages of trimeric complexes. Such solution was eventually found under the form of a single-chain version of the trimer, wherein the C-terminal end (C-terminus) of a first constituting alpha-helix is connected (joined, linked) to the N-terminal end (N-terminus) of a second alpha-helix, and the C-terminal end of the latter to the N-terminal end of a third alpha-helix. The latter connections are realized through the usage of a structurally flexible linker fragment.

Flexible linker fragments are frequently used in the field of protein engineering to interconnect different functional units, e.g. in the creation of single-chain variable fragment (scFv) constructs derived from antibody variable light (VL) and variable heavy (VH) chains. At present, the application of flexible linker fragments in combination with trimeric coiled coil structures, for the purpose of creating a single-chain yet triple-stranded coiled coil scaffold structure has not been disclosed nor anticipated in the public domain. It is also remarked that there is no contradiction in the formulation 'single-chain triple-stranded' because 'single-chain' refers to the full amino acid sequence, whereas 'triple-stranded' is the common term to denote that the coiled coil structure consists of three individual alpha-helical strands (chain fragments).

The present invention relates to a single-chain yet triple-stranded alpha-helical coiled coil protein scaffold having a full amino acid sequence of the formula HRS1-L1-HRS2-L2-HRS3, wherein HRS1, L1, HRS2, L2 and HRS3 represent amino acid sequence fragments that are covalently and consecutively interconnected in the order as indicated in the said formula, and wherein

a) the three fragments HRS1, HRS2 and HRS3 are composed or consist of heptad repeat sequences (of 7 amino acid residues) of formula abcdefg, and
b) fragments L1 and L2 are structurally flexible linker sequences;

and wherein the full amino acid fragments spontaneously and stably folds - in solution by way of these HRS1, HRS2 and HRS3 fragments - into a triple-stranded, parallel, alpha-helical coiled coil structure.

Another aspect of the present invention relates to a single-chain coiled coil scaffold wherein these heptad repeat sequences comprise at least two consecutive (7-residues) heptad repeat units.

Another aspect of the present invention relates to this single-chain coiled coil scaffold wherein at least 50% of the heptad a-positions of these heptad repeat sequences are occupied by isoleucines, because of the intrinsic propensity of isoleucine amino acids to form trimeric coiled coils.

Another aspect of the present invention relates to this single-chain coiled coil scaffold wherein at least 70% of the heptad a-positions of these heptad repeat sequences are occupied by isoleucine amino acids.

A further preferred embodiment of the present invention relates to this single-chain coiled coil scaffold wherein at least 90% of the heptad a-positions are occupied by isoleucine amino acids.

Another aspect of the present invention relates to this single-chain coiled coil scaffold wherein all heptad a-positions of these heptad repeat sequences are occupied by isoleucine amino acids except scaffolds wherein these fragments comprise more than two consecutive (7-residue) heptad repeat units (because if all a-positions are occupied by isoleucines there exists a risk of misfolding (e.g., heptad register shifts or aggregation)).

Another aspect of the present invention relates to this single-chain coiled coil scaffold wherein one or more of the heptad a-positions of these heptad repeat sequences are occupied by amino acids selected from the group consisting of valine, leucine, methionine, phenylalanine, tyrosine, tryptophan, histidine, glutamine, threonine, serine, alanine, or glycine, because such amino acid types occasionally (infrequently) appear at these a-positions in natural trimeric coiled coils.

Another aspect of the present invention relates to this single-chain coiled coil scaffold wherein one or more of the heptad a-positions of these heptad repeat sequences are occupied by amino acids selected from the group consisting of valine, leucine, methionine, because such amino acid types regularly appear at these a-positions in natural trimeric coiled coils.

According to the present invention the flexible linker sequences have an amino acid composition which is made of at least 70% of the (comprising at least 70%) amino acids selected from the group consisting of glycine, serine, threonine, alanine, proline, histidine, asparagine, aspartic acid, glutamine, glutamic acid, lysine, or arginine. These amino acid are largely polar and are therefore well-suited to be integrated in a structurally flexible linker fragment, because polar amino acids have a greater propensity to become solvated and, hence, form a lower risk to interact within the scaffold in an unintended manner.

Another aspect of the present invention relates to this single-chain coiled coil scaffold wherein the flexible linker sequences have an amino acid composition which is made of at least 70% of (comprising at least 70%) amino acids selected from the group consisting of glycine, serine, threonine, alanine, or proline. These amino acids are largely polar and small and form a minimal risk to interact within the scaffold in an unintended manner.

Another aspect of the present invention relates to this single-chain coiled coil scaffold wherein the flexible linker sequences have an amino acid composition which is made of at least 70% of (comprising at least 70%) amino acids selected from the group consisting of glycine, or serine.

These amino acids are frequently used in flexible linker fragments.

Another aspect of the present invention relates to this single-chain coiled coil scaffold wherein the flexible linker sequences have an amino acid composition comprising only glycine and/or serine. These amino acid types are frequently used in flexible linker fragments.

Another aspect of the present invention relates to this single-chain coiled coil scaffold wherein the number of amino acid residues of each of the linker sequences accounts for at least half (at least 50%) of the number of amino acid residues within the coiled-coil forming region of the two heptad repeat sequences that are linked by these respective linker sequences. The distance in 3-D space from one end of a heptad repeat sequence to the beginning of the next heptad repeat sequence can be roughly calculated by the formula 'number of residues in the coiled coil region multiplied by 1.5 Angstrom'. The distance that can be obtained (bridged) by a linker in extended conformation can be roughly calculated by the formula 'number of residues in the linker fragment multiplied by 3.0 Angstrom'. Hence, the linker must have at least half (at least 50%) of the number of coiled coil residues to obtain (to enable) connection in a relaxed manner. Such relaxed connection is a most preferred feature to facilitate correct (intended) folding.

Another aspect of the present invention relates to this single-chain coiled coil scaffold wherein amino acid residues near the termini of each linker sequence are selected to (such that they) stabilize the helical ends of this coiled coil region. Such stabilization of the helical ends is generally known in the art as 'helical capping'. Proper helical capping is a most preferred feature to facilitate correct (intended) folding whereby the resultant scaffold still fulfills all features as recited in claim 1.

The present invention relates to this single-chain coiled coil scaffold wherein amino acid residues near the termini of each linker sequence are selected to promote formation of a turn in between the connected alpha-helix and the remainder of the linker. Since a linker has to bridge between two distal ends of a coiled coil region (in view of the parallel nature of the constituting alpha-helices), each linker must necessarily make a turn (reversal of the chain direction) at either end. Proper selection of turn-forming amino acid types is therefore highly preferred. The turn-promoting amino acid types are selected from the group consisting of proline and glycine, asparagine and aspartic acid.

Another aspect of the present invention relates to this single-chain coiled coil scaffold wherein amino acid residues at conventional heptad e- and g-positions are selected to (such that they) promote the stability of this scaffold, because a high stability of the scaffold is a highly preferred feature of any type of scaffold. Heptad e- and g-positions in spatially adjacent alpha-helices are oriented towards each other, which makes them inherently suited for stability engineering purposes.

Another aspect of the present invention relates to this single-chain coiled coil scaffold which remains thermodynamically stable under physical conditions that differ by at least 2 pH units (preferably 4 pH units), 20°C (preferably 40°C) and/or a factor 2 (preferably a factor 4) in ionic strength from physiological conditions being a pH of about 7, a temperature of about 37 °C and an ionic strength of about 0.15 molar. Such scaffold is highly preferred, because of its robustness and tolerance to changes in the conditions (particular, occasionally extreme, physical, biophysical or physiological conditions) under which the scaffold is applied.

Another aspect of the present invention relates to this single-chain coiled coil scaffold wherein wherein amino acid residues at conventional heptad b-, c- and f-positions are selected such that they promote the solubility of this scaffold. A highly soluble scaffold is highly preferred, because the active (functional) state will generally be the soluble state. Since heptad b-, c- and f-positions are solvent-oriented in a trimeric coiled coil, these positions are ideally suited to be occupied by solubility-promoting amino acid residue types. Such solubility-promoting residue types include, typically, polar residue types.

Another aspect of the present invention relates to this single-chain coiled coil scaffold which remains soluble in aqueous solutions up to a concentration of about 1 micromolar (preferably about 1 millimolar). A solubility (i.e., the concentration at which a given compound starts precipitating) exceeding about 1 micromolar (preferably about 1 millimolar) is highly preferable because it guarantees that the compound remains soluble at concentrations at which it is typically applied.

Another aspect of the present invention relates to this single-chain coiled coil scaffold wherein amino acid residues at conventional heptad g-positions in the heptad repeat sequence HRS1 and at conventional heptad e-positions in the heptad repeat sequence HRS3 form a structural advantageous indentation, or groove, into the surface of this scaffold. The presence of a (concave) groove in the surface of a scaffold is highly preferred, for it provides a 'predestined' (ideally suited) region on the surface to mediate binding of structurally complementary (typically, convex) ligand molecules (proteins, peptides, DNA molecules, polysaccharides, ions, etc). In view of the geometric properties of a trimeric coiled coil, each pair of adjacent alpha-helices displays a more or less pronounced groove that runs parallel with, and in between, these alpha-helices. The depth of a groove, and its physical characteristics, mainly depends on the presence of (selection of) specific amino acid residues at the e- and g-positions.

Another aspect of the present invention relates to this single-chain coiled coil scaffold wherein conventional heptad g-positions in the heptad repeat sequence HRS1 and conventional heptad e-positions in the heptad repeat sequence HRS3 are occupied by amino acid residues of which the backbone moieties carry side chains that participate in the interface of a molecular complex between this scaffold and a target molecule. A highly valuable application of the present invention relates to scaffold molecules that bind to a given target molecule, e.g., a therapeutically important target molecule. Since heptad e- and g-positions are geometrically favorably located (oriented) to mediate binding, i.e., to participate in the interface between the scaffold and the target, a proper selection of amino acid types at these positions will be critical for the binding (binding strength and specificity). Useful methods for selecting interface residues at e- and g-positions include, without limitation, library-based methods, phage-display methods, directed evolution methods, rational or semi-rational design methods (possibly including molecular modeling techniques), etc. In a preferred embodiment, g-positions from HRS1 and e-positions from HRS3 will be examined, because the left-handed supercoiling of the coiled coil scaffold, in combination with a (properly chosen ('not too long')) linker fragment, logically (though not obligatory) results in a binding groove wherein g-positions in HRS1 and e-positions in HRS3 constitute the major part of the interface surface.

Another aspect of the present invention relates to this single-chain coiled coil scaffold wherein conventional heptad g-positions in the heptad repeat sequence HRS1 and conventional heptad e-positions in the heptad repeat sequence HRS3 are occupied by amino acid residues that are selected to (such that they) optimize the binding of the said scaffold to a target molecule. A highly preferred embodiment of the present invention is this scaffold which shows optimal binding characteristics to a target molecule, wherein 'optimal binding' may relate to binding strength (affinity) and/or specificity (discriminatory power, selectivity). Alternatively, in some applications, scaffold molecules will be desired with optimized binding kinetics. In specific applications, scaffold molecules will be desired with optimized avidity. In all of these applications, it may be highly preferred to optimize the binding with respect to the intended characteristic. In view of their geometrical properties, e- and g-positions (but possibly also other positions) will in such cases, preferably be selected for optimization. Specific optimization approaches may include, without limitation, library-based methods, phage-display methods, directed evolution methods, rational or semi-rational design methods (possibly including molecular modeling techniques), alanine-scanning methods, maturation methods, etc.

Another aspect of the present invention relates to this single-chain coiled coil scaffold wherein amino acid residues participating in the interface of a molecular complex between this scaffold and a target molecule are optimized to such extent that the molecular complex has a dissociation constant Kd lower than about 1000 micromolar, preferably lower than about 100 micromolar, more preferably lower than about 10 micromolar or most preferably lower than about 1 micromolar.

The binding strength (affinity) of a given molecule (e.g., a scaffold molecule of the present invention) for another molecule (ligand, e.g. a pharmaceutically relevant target molecule) is commonly expressed in terms of the dissociation constant (Kd) of this complex between the two molecules.

Binding affinity is inversely related to the dissociation constant (the lower Kd, the higher the affinity). Although some detection methods (assays) may have a sensitivity (detection limit) for Kd's higher than about 1000 micromolar (1 millimolar), preferably the single-chain coiled coil scaffold described in the present invention relates to scaffold molecules having a Kd lower than about 1000 micromolar. Preferably, however, the Kd will be lower than about 100 micromolar, more preferably lower than about 10 micromolar and most preferably lower than about 1 micromolar, the latter being a Kd that is often considered as corresponding to an affinity that is 'acceptable'.

A scaffold molecule having a Kd in the range 1-1000 micromolar will also be valuable, because it can be submitted to one or more rounds of affinity maturation.

There exist many possible technical methods to generate this single-chain scaffold molecule comprising a 3-stranded, parallel, alpha-helical coiled coil structure, which displays specific, desired binding properties. Such methods may include a variety of state-of-the-art experimental and theoretical (i.e., bioinformatics and molecular modeling) methods, and combinations thereof.

In principle, the majority of methods that were previously proven useful to generate target-specific immunoglobulin- or protein scaffold-derived molecules should also be useful for the design of single-chain coiled coil structures. In addition to these state-of-the-art methods, the following non-standard method is provided as well; this method exploits some of the characteristics that are unique to 3-stranded coiled coils (e.g., the availability of structural information in the Protein Data Bank, the trimeric nature of coiled coil structures, experimental data on protein fragments in association with trimeric coiled coils, and the like) and is therefore considered a preferred, though not obligatory, design method.

Concretely, a method to generate a specific scaffold molecule of the present invention, displaying detectable binding to a given target molecule includes the steps of

(step 1) providing a reference 3-D structural representation of a triple-stranded, parallel, alpha-helical coiled coil complex by retrieving corresponding atomic coordinates from a Protein Data Bank;

(step 2) providing a set of 3-D structural representations of triple-stranded, parallel, alpha-helical coiled coil complexes that are associated with additional proteinaceous elements, by retrieving corresponding atomic coordinates from a Protein Data Bank;

(step 3) superimposing one or more of the obtained representations of step (2) onto the obtained representation of step (1) by applying a least-squares fitting method to a portion or all of the backbone atoms of coiled coil regions of representations of steps (1) and (2);

(step 4) selecting, from coiled coil regions of representations of step (2), amino acid residues that are in contact with associated additional proteinaceous elements;

(step 5) in silico grafting, by way of transferring atomic coordinates, either the entire amino acid residues selected in step (4), or only their side chains, onto the reference 3-D structural representation of step (1), and, optionally, optimizing conformation of grafted amino acid residues by using a suitable molecular modeling method, for example, an energy minimization method or a molecular dynamics method;

(step 6) selecting, from grafted reference structure of step (5) a trimeric coiled coil region which at least encompasses grafted amino acid residues;

(step 7) obtaining two, possibly identical, suitable linker fragments, (e.g. linker fragments that are rich in glycine and serine) having a number of amino acid residues which amounts at least half (at least 50%) of the number of amino acid residues within the coiled-coil region selected in step (6).

(step 8) optionally, selecting useful helix-capping residues near termini of these linker fragments;

(step 9) obtaining a final, linear, amino acid sequence construct that includes the selected coiled coil fragments comprising the grafted amino acid residues, the coiled coil fragments being interspaced by the linker fragments selected in step (7) and optionally provided with capping residues selected in step (8);

(step 10) obtaining a gene construct encoding for the linear amino acid sequence of step (9), synthesizing this gene construct, producing the amino acid sequence (protein) using a suitable expression system of a cell (e.g., *E. coli*), and isolating and purifying the protein from contaminants;

(step 11) optionally, performing biophysical tests on the protein obtained in step (10)and

(step 12) measuring a binding affinity of the protein obtained in step (10) for one or more ligand molecules comprising the additional proteinaceous elements identified in step (2).

As will be acknowledged by persons skilled in the field of molecular modeling and, by extension, protein design, not all theoretically binding ligand molecules will be confirmed as such in experimental binding assays. Yet, theoretical methods (e.g., steps 1 - 8 of said workable method) can considerably expedite identification of novel binders by formulating a limited number of potential binders that can be experimentally tested (e.g., in step 12 of said workable method). In addition, low-affinity scaffold molecules can be submitted to additional rounds of affinity maturation using conventional approaches.

The discovery of an initial binder to a particular target of interest does not necessarily imply that this binder will become a true drug. Almost invariably, initial hits have to be modified (i.e., changed in 'chemical space' for small-molecule compounds or in 'sequence and chemical space' for proteinaceous compounds) in order to improve, on the one hand, affinity and specificity and, on the other hand, solubility, permeability and metabolic stability.

Such modifications are generally performed in downstream steps of drug development processes. Correspondingly, chemical and/or structural modifications to the scaffold molecules of the present invention do not form explicit embodiments of the present invention itself.

In addition, the scaffold of the present invention should in principle be amenable to a vast number of modifications including (multiple) amino acid substitutions, introduction of non-natural amino acids, attachment of particular chemical moieties, peptidic extensions, labeling, avidity enhancement through self-concatenation, concatenation into fusion proteins, etc., without compromising (changing, destroying) the scaffold itself as long as the resultant scaffold still fulfills all features of claim 1.

A number of such modifications can be formulated here to illustrate the intrinsic potential of the scaffold to be subject to advanced engineering steps.

Concretely, the present inventors contemplate the following engineered constructs, which all include the scaffold of the present invention with all of its specified characteristics:

- any scaffold of the present invention may be modified in amino acid sequence, thereby creating one or more derivatives thereof;
- any scaffold or derivative may be modified, e.g., to enhance its stability;
- any scaffold or derivative may be modified, e.g., to enhance its folding kinetics;
- any scaffold or derivative may be modified, e.g., to enhance the correctness of its folded state;
- any scaffold or derivative may be modified, e.g., to enhance its binding affinity to a target compound;
- any scaffold or derivative may be modified, e.g., to enhance its binding specificity for a target compound;
- any scaffold or derivative may be modified, e.g., to enhance its solubility;
- any scaffold or derivative may be covalently linked to any other protein or proteinaceous molecule, either via its N- and/or C-terminal ends or via one or more of its side chains, such covalent linkage being reminiscent of the fundamental characteristic of a scaffold as a 'carrier of substituents';
- any scaffold or derivative may be covalently linked to other copies of the same scaffold or derivative, e.g., to increase avidity;
- any scaffold or derivative may be covalently linked to any scaffold or derivative with different binding properties, e.g., to provide bi- or multispecificity;
- any scaffold or derivative may be covalently linked to any existing natural or non-natural protein or protein domain or peptide that is not related to the present invention, including, without limitation, Fc domains, Fc receptor, serum albumin, fluorescent proteins, scaffold molecules of another type, etc.;

- any scaffold or derivative may be covalently linked to one or more detection tags;
- any scaffold or derivative may be covalently linked to one or more purification tags;
- any scaffold or derivative may be covalently linked to organic compounds by way of a chemical reaction with one or more scaffold side-chain moieties;
- any scaffold or derivative may be glycosylated;
- any scaffold or derivative may be PEGylated as long as the resultant scaffold fulfills all features of claim 1.

In view of the fact that the scaffold of the present invention, and derivatives thereof, form stable and compact structures, they may be constructed or manipulated for research purpose or for improving their industrial applications in principle, by all techniques applicable to proteins and protein scaffolds.

While many of the aforementioned handlings known in the field of protein engineering may appear either highly advanced or straightforward, depending on the level of expertise of the person confronted, none of them can be applied without taking the proper precautions. Therefore, such constructs will need to be investigated further. They do not form embodiments of the present invention, although the basic physical and technical principles are disclosed here.

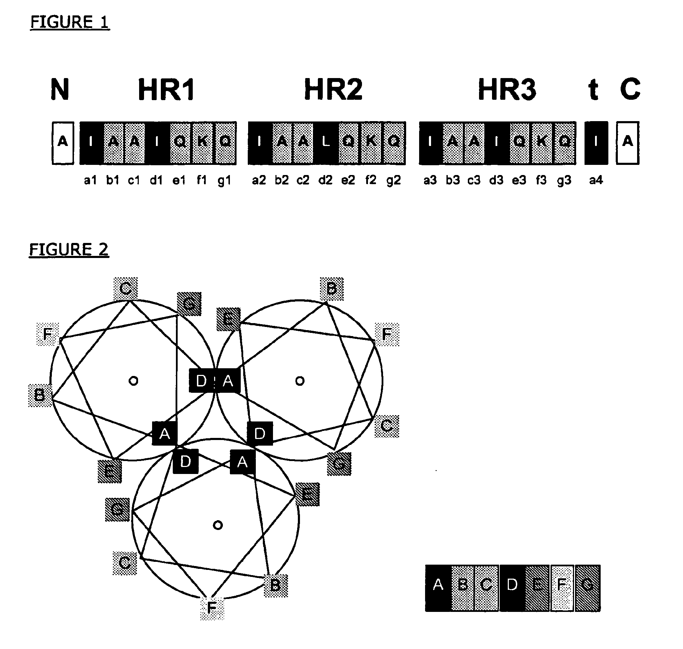

Heptad core residues are shielded from solvent in triple-stranded, parallel, alpha-helical coiled coil complexes, as illustrated in Figure 2. Non-covalent interactions between contacting core residues (positions A and D in Figure 2) provide the main thermodynamic driving force for the peptides to adopt such fold. The Figure 2 is a helical wheel representation of a triple-stranded, parallel, alpha-helical coiled coil scaffold. The left panel shows a top view on the scaffold. The right panel shows a linear sequence of heptad repeat positions. Only one heptad repeat is displayed for clarity reasons. Different shades are used to indicate specific topological positions. The non-core residues (positions B, C, E, F and G) are at least partially solvent-accessible (positions E, G less than B, C, and positions B, C less than F) and are susceptible to amino acid substitutions without (major) implications for the stability of the complex (scaffold structure).

## EXAMPLES

Example 1. Amino acid sequence of an artificial peptide with core and non-core residues.

This example provides the amino acid sequence of a specific peptide forming an instance of the present invention. The amino acid sequence, AIAAIQKQIAALQKQIAAIQKQIA, is presented in single-letter notation, wherein A refers to alanine, I to isoleucine, L to leucine, Q to glutamine, and K to lysine. The peptides with this amino acid sequence fold in solution into triple-stranded, parallel, alpha-helical coiled coil complexes by way of their isoleucine and leucine amino acid residues forming a hydrophobic core (the center or the interior) and the other residues being oriented towards solvent. The artificial peptide comprises three heptad repeats labeled "HR1", "HR2" and "HR3" as presented in Figure 1.

The Figure 1 is a schematic representation of the amino acid sequence of an artificial peptide comprising heptad repeats (HRx), core residues (black boxes), non-core residues (gray boxes) and flanking regions (white boxes). The peptide further comprises a C-terminal heptad core residue labeled "t". The peptide further comprises N- and C-terminal flanking fragments labeled "N" and "C", respectively. Each heptad repeat residue is further annotated with indices "a" to "g" and a number corresponding to the heptad repeat number. Core residues are located at a- and d-positions. It is seen that 5 out of the 6 core residues of the three full heptad repeats are isoleucines. The isoleucine residue labeled "a4" belongs to the partial heptad repeat "t".

Example 2. Alpha-helical structure and reversible folding/unfolding.

Peptidic alpha-helical coiled coils do not form the subject of the present invention, because they do not fold into a single-chain scaffold. However, the single-chain scaffold of the present invention does comprise a trimeric coiled coil region. Evidently, connecting the N- and C-terminal ends by linker fragments can (will) influence the folding kinetics, but the essential physical properties of the 'excised' coiled coil peptides are expected to be generally preserved. Hence, peptidic coiled coils may serve as a study system, for research purpose.

To demonstrate quantitative formation of alpha-helical secondary structure of a reference artificial peptide in solution, the inventors have synthesized the peptide with the amino acid sequence Ac-MSIEEIQKQQAAIQKQIAAIQKQIYRMTP-NH2 and recorded the circular dichroism (CD) spectrum. The amino acid sequence is given in single-letter code; Ac- and -NH2 mean that the peptide was acetyl-initiated and amide-terminated, respectively.

This peptide is to be considered as a derivative of the reference peptide composed of the triple heptad repeat sequence (IAAIQKQ)3, with modifications at the amino- (N-) and carboxy- (C-) terminal ends to improve the alpha-helical nature of the termini (often referred to as capping).

More specifically, the flanking residues Ac-MS- were attached at the N-terminus, in combination with the substitution of two consecutive glutamic acid residues (EE) for the two alanine residues (AA) in the first heptad of the reference sequence.

Furthermore, the flanking residues -IYRMTP-NH2 were attached at the C-terminus, such that the amino acids isoleucine (I) and methionine (M) are located at conventional heptad a- and d-positions, allowing this flanking sequence to form an extra, though incomplete, heptad. The tyrosine (Y) was introduced at a solvent-oriented b-position to enable spectrophotometric concentration determination. The arginine (R), threonine (T) and proline (P-NH2) residues were introduced to improve C-terminal helical capping. In addition, the isoleucine (I) residue at the a-position of the second heptad was replaced by a glutamine (Q) residue to force the coiled coil-forming peptides to associate in the correct (intended) way, i.e., to ascertain formation of a trimeric complex and to avoid possible heptad register shifts [Eckert et al. J Mol Biol 1998, 284:859-865].

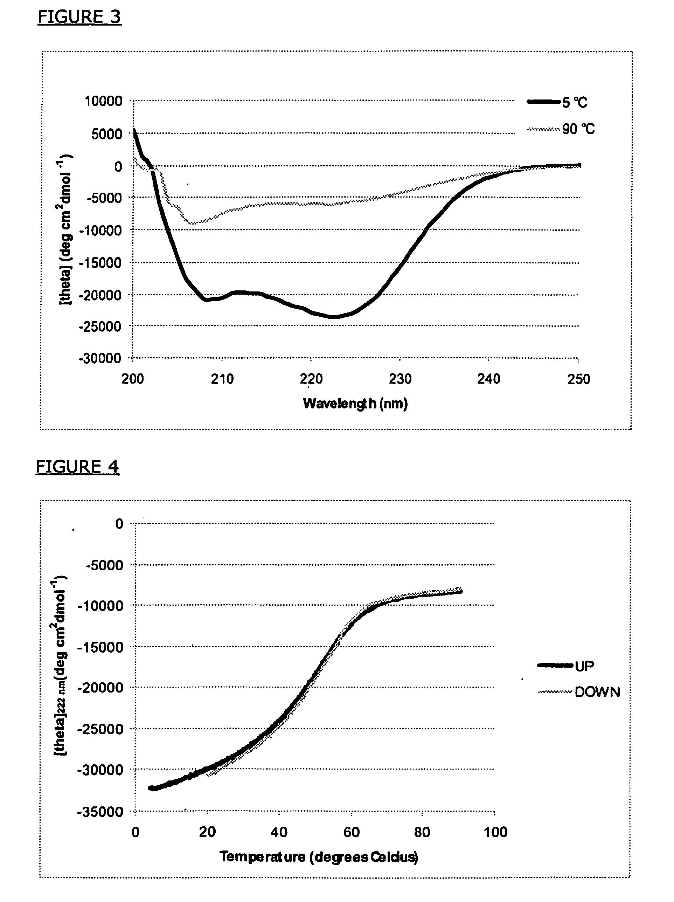

The said synthesized peptide was dissolved at a concentration of 292 microM in 20 mM phosphate buffer (PBS), 150 mM NaCl, pH 7.2. The CD spectra were measured between 200 and 250 nM, at 5 and 90 degrees Celsius (Figure 3). The spectrum at 5 degrees Celsius was indicative of a high alpha-helical secondary structure content, in agreement with the expectation that all heptad regions, but not all of the flanking residues, would assemble as alpha-helical coiled coils. The spectrum at 90 degrees Celsius showed that the alpha-helical structure was greatly, but not completely, lost at elevated temperatures.

To analyse whether the temperature-induced transition between helical and nonhelical states was reversible, a forward (up) and backward (down) thermal scan was performed on the same sample, by recording the CD signal at 222 nM as a function of temperature at a scanning rate of about 1 degree Celsius per minute (Figure 4). It was observed that the up and down scans almost perfectly coincided, thereby confirming the quantitative unfolding and refolding of the peptides in the sample.

It was further analyzed whether the thermal unfolding curve of Figure 4 conformed to the thermodynamic equations describing the equilibrium folding/unfolding reaction between three molecules free (monomeric) peptide and one entity of folded (trimeric) complex. This reaction is generally written as

3 peptide <=> peptide$_3$

wherein "<=>" refers to a chemical equilibrium, "peptide" to a monomeric peptide in solution and "peptide$_3$" to a trimeric entity in the folded (assembled, associated) state. This thermal unfolding curve was fitted to the theoretic equations:

$$\theta(T) = \theta_M(T) + (\theta_T(T) - \theta_M(T))\left(1 + \sqrt[3]{F\left(-\frac{1}{2} + \sqrt{\frac{1}{4} + \frac{F}{27}}\right)} + \sqrt[3]{F\left(-\frac{1}{2} - \sqrt{\frac{1}{4} + \frac{F}{27}}\right)}\right)$$

wherein

$$F = \frac{\exp\left(-\frac{\Delta H_t}{RT}(1 - T/T_t) - \frac{\Delta C_p}{RT}(T - T_t - T\ln(T/T_t))\right)}{4}$$

and

$T \equiv$ the temperature, in degrees Kelvin, of the sample

$\theta(T) \equiv$ the CD-signal [theta]$_{222\,nm}$, in deg cm$^2$ dmol$^{-1}$, as a function of $T$

$\theta_M(T) \equiv$ the CD-signal for 100% free (monomeric) peptide as a function of $T$

$\theta_T(T) \equiv$ the CD-signal for 100% associated (trimeric) peptide as a function of $T$

$T_t \equiv$ the transition temperature, where 50% of the total peptide concentration is associated

$\Delta H_t \equiv$ the enthalpy difference, in kJ per mole peptide, between mono- and trimeric states

$\Delta C_p \equiv$ the heat capacity difference, in J mol$^{-1}$ K$^{-1}$, between mono- and trimeric states

$R \equiv$ the ideal (universal) gas constant $\equiv 8.31$ J mol$^{-1}$ K$^{-1}$

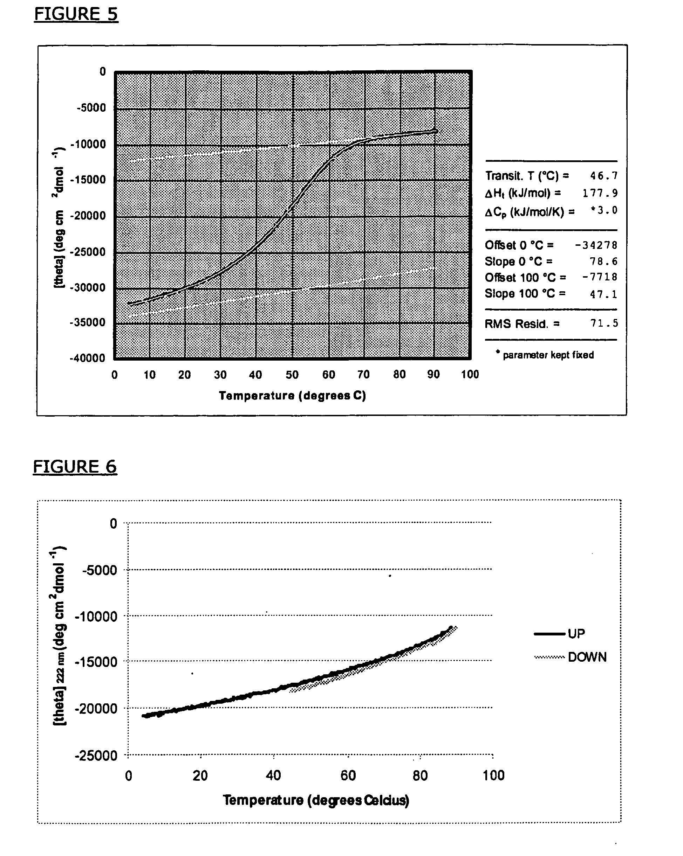

The results of this fitting operation are shown in Figure 5. It was found that the theoretic curve almost perfectly coincided over the entire temperature range with the experimental curve, thereby confirming trimeric association of the peptides.

Figure 5 represents fitting of a theoretic equation for trimeric association to experimental data. The experimental data are taken from Figure 4, curve labeled "UP". The theoretic equations used are listed *supra.* The fitted parameters (fitting results) are listed at the right in Figure 5. "Transit. T" corresponds to $T_t$, but is expressed here in degrees Celsius. The parameter "delta C$_p$" was kept constant at 3.0 kJ mol$^{-1}$ K$^{-1}$. The parameters "theta$_M(T)$" and "theta$_T(T)$" were treated as linear functions of T, resulting in the white straight lines described by the respective offsets and slopes indicated at the right in the figure 5. "RMS Resid." refers to the root-mean-square of the differences between experimental and theoretic data points. The fitted (theoretic) curve itself is plotted in white on the figure 5 and coincides over the entire temperature range with the experimental data points shown in black.

Example 3. Usage of all-isoleucine core residues.

To analyse whether the glutamine residue at position a of the second heptad in the reference peptide of Example 2 was required for correct (intended) folding into a trimeric coiled coil, this residue was replaced by isoleucine, resulting in a peptide named "Q2aI" having a sequence with isoleucine at all core positions (except methionine within the C-terminal flanking fragment). For this purpose, the peptide with the following sequence was synthesized: Ac-MSIEEIQKQIAAIQKQIAAIQKQIYRMTP-NH2.

Figure 6 shows the thermal denaturation curve for a sample preparation of the Q2aI peptide under the same conditions as in Example 3. The global CD signal was somewhat lower than expected, which could be due to an instrumental deviation, an error in the concentration determination, a lower purity, or a lower than expected alpha-helical content. Nevertheless, the main goal of this experiment was to examine the effect of the glutamine-to-isoleucine mutant on the stability of the complex. It was therefore interesting to find that this variant showed extremely high resistance against thermal denaturation, i.e., it was extremely thermostable. The estimated transition temperature was around 97 degrees Celsius, although the latter was difficult to determine because of incompleteness of the transition. Also, the down-scan showed full recovery of the CD signal, indicating full reversibility.

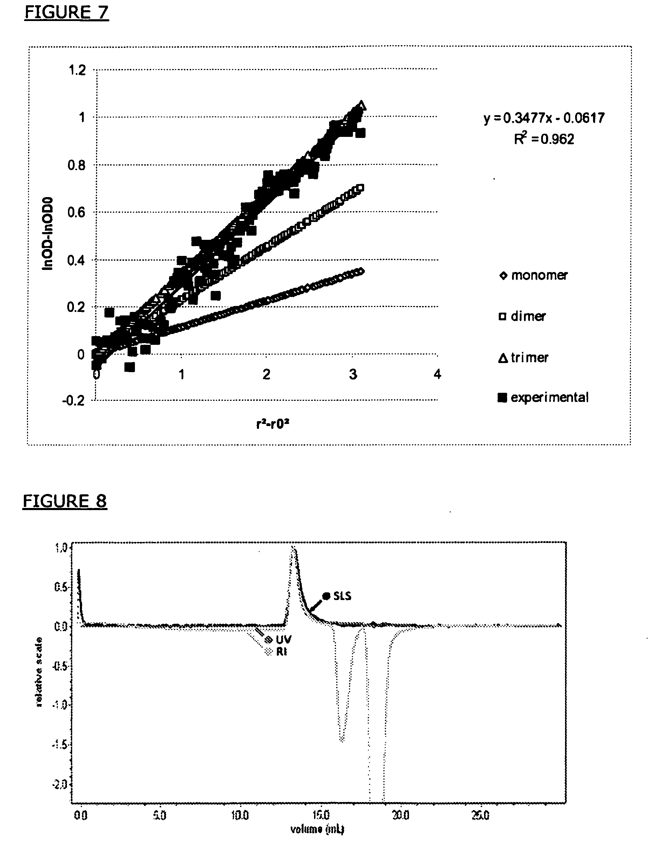

To confirm that the assembled complex had the correct molecular weight (MW), as expected for a trimer, the Q2aI peptide was submitted to analytical sedimentation equilibrium ultracentrifugation at 25000 rpm at a concentration of approximately 1 mg/ml. Figure 7 shows the linearized optical density (OD) curve in comparison with the theoretical curves for monomeric, dimeric and trimeric complexes. It was found that the experimental data points coincided very well with the trimeric model curve. From the slope of the linear regression line, the apparent molecular weight of 10500 Da was derived, in good agreement with the theoretic value of 10242 Da (3 times the MW of 3414 Da for a monomer).

To further confirm formation of trimeric complexes, the same Q2aI peptide was also analyzed by static light scattering. 200 microliter peptide at 1 mg/ml in PBS was put on a Superdex 75 10/300 GL gel filtration column connected to ultra-violet (UV), refractive index (RI) and static light scattering (SLS) detectors. Figure 8 shows the results. The signals (curves) from the three different detectors are labeled accordingly. A well-shaped light scattering peak was observed coinciding with a UV and RI peak. The apparent molecular weight derived for the UV peak was 12530 ± 1510 Da, again in good agreement with the expected value.

It was concluded that the usage of all-isoleucine core residues did not have an adverse effect on the assembly of the peptide into trimers, as could be expected on basis of theoretic considerations about potential (unintended) heptad register shifts. Instead, all tests indicated the proper and exclusive folding into trimers with the correct (expected) molecular weight. Furthermore, this all-isoleucine core peptide had a very high thermal stability, for it did not quantitatively unfold up to 95 degrees Celsius. Therefore, this peptide can be considered as a preferred trimeric coiled coil-forming scaffold peptide.

Example 4. Analysis of core mutants.

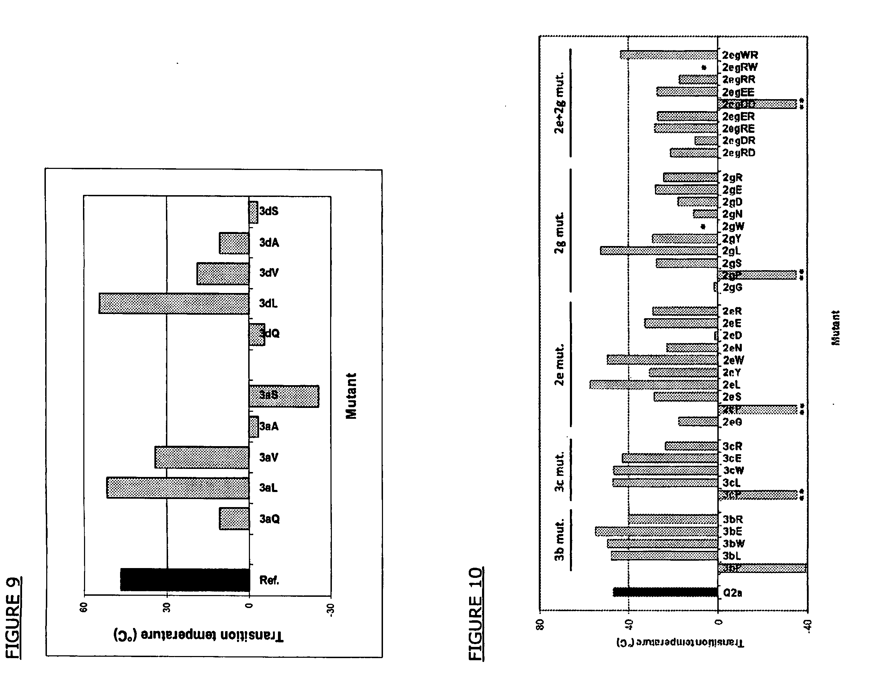

To further analyse the tolerance of the trimeric coiled coil scaffold towards amino acid residue substitutions at specific core positions, the following mutants of the reference peptide examined in Example 3 (i.e., the one comprising a glutamine at position 2a, not the more stable Q2aI mutant of Example 4) were synthesized: 3aQ, 3aL, 3aV, 3aA, 3aS, 3dQ, 3dL, 3dV, 3dA and 3dS, wherein each time "3" refers to the third heptad and "a" and "d" refer to a- and d-positions, respectively; the letters Q, L, V, A and S refer to mutations into glutamine, leucine, valine, alanine and serine, respectively. All ten peptides were analyzed by thermal CD scanning as in Examples 3 and 4. Fitting of the thermal denaturation curves, as described in Example 3, gave the corresponding transition temperatures shown in Figure 9. It was found that, compared to the reference peptide comprising isoleucine at positions 3a and 3d, only leucine had a slightly stabilizing effect at both the a- and d-positions (3aL and 3dL, respectively). All other substitutions had moderately (I3aV) or relatively strong (I3dV, I3aQ and I3dA) destabilizing effects or were even disruptive (I3aA, I3aS, I3dQ and I3dS).

The observation that leucine appears to be a well-tolerated core substitution was somewhat unexpected in view of the general idea that leucines are preferred in so-called dimeric "leucine zippers" [Harbury et al. Science 1993, 262:1401-1407]. Nevertheless, our observation that valine is tolerated better at a- than at d-positions is in line with the findings of Harbury et al. [ibid].

The additional mutants having glutamine, alanine or serine at core positions are all markedly unstable. These findings can be further extrapolated to other possible core substitutions: in view of the chemical equivalence between leucine and methionine, and the presence of a methionine at the C-terminal core position in our reference peptide, it can be expected that this residue type, together with leucine and the "standard" isoleucine, and to a lesser extent, valine, are tolerated at core positions. Other amino acid residue types are shown and expected to be highly destabilizing. Further, in view of the fact that non-isoleucine core residues increase the propensity of the peptides to form oligomers other than trimers [Harbury et al. Science 1993, 262:1401-1407], isoleucine is a highly preferred core residue. Leucine, methionine and, to a lesser extent, valine, are less preferred core residues. All other residue types are non-preferred core residues.

Example 5. Analysis of non-core mutants.

To determine whether or not trimeric coiled coil scaffolds are tolerant to substitutions of non-core residues, various single and double mutants were made of the reference peptide of Example 3. CD thermal scans were recorded and analyzed in the same way as in Examples 3-5.

The amino acid proline is known to be structurally disruptive in alpha-helices. Glycine has a very low alpha-helical propensity but is generally not disruptive. All other residue types are compatible with alpha-helices but each have a different helical propensity. Moreover, it has been demonstrated that particular non-core residues, especially at e- and g-positions can significantly influence the stability of a coiled coil, at least in dimers but likely also in trimeric complexes [Yu Adv Drug Deliv Rev 2002, 54:1113-1129].

For the purpose of this example, we selected and introduced a representative number of residue types at non-core positions and tested their effect on the stability of the trimeric scaffold structure formed by the reference peptide of Example 3, denoted as "Q2a". It would be useful to test these mutations within the context of the thermally stable Q2aI mutant of Example 4 (having an all-isoleucine core) in order to demonstrate tolerance of this preferred scaffold to a variety of amino acid substitutions, such tolerance being a required property of a useful scaffold. However, the latter experiments would likely prevent quantitative determination of stability effects (see Example 3).

Therefore, the moderately stable reference peptide Q2a (with glutamine at core position 2a) was selected as a practically more suitable context. In view of the fact that core and non-core residues are spatially separated in a coiled coil structure (see Example 2), there is little or no doubt that at least the general experimental observations apply to coiled coils with different core substitutions. For the purpose of this example, leucine (L), tryptophan (W), glutamic acid (E) and arginine (R) were selected as representative aliphatic, aromatic, negatively charged and positively charged amino acids, respectively; the latter amino acids should preferably be tolerated by the scaffold.

In addition, the "control" amino acid proline was selected, which should be disruptive if the scaffold structure folds in the way as intended.

The said amino acid residue types were substituted at the non-core positions b and c of the third heptad (i.e., at positions 3b and 3c) in peptide Q2a. The list of selected amino acids was further amended for testing mutability at e- and g-positions in the second heptad (i.e., at positions 2e and 2g) in peptide Q2a.

At these positions, the amino acids glycine (G), serine (S), tyrosine (Y), asparagine (N) and aspartic acid (D) were also tested. In addition, certain combination mutants of tryptophan, aspartic acid, glutamic acid and arginine at e- and g-positions in the second heptad of peptide Q2a were also tested in order to examine additivity of point mutations.

Figure 10 shows the experimentally determined transition temperatures (bars) for the tested point and double mutants. The mutants are denoted by the position of the mutation followed by the mutant residue type in single-letter notation (for example, "3bL" indicates leucine at position 3b). Double mutants are denoted by the modified heptad, followed by the modified positions, followed by the mutant single-letter residue types at the respective positions (for example, "2egRD" indicates arginine at 2e and aspartic acid at 2g). Mutants labeled by a single asterisk (*) were not determined because they could not be synthesized to sufficient purity. Mutants labeled by a double asterisk (**) were highly unstable, having transition temperatures presumably below -35 °C, and the corresponding values were fixed at this value.

Compared to the reference sequence of Q2a (carrying alanine at positions 3b and 3c and glutamine at 2e and 2g), it is firstly seen that there are more destabilizing than stabilizing mutations, suggesting that the chosen reference sequence is one of the more preferred (with Q2aI being an even more preferred sequence, see Example 4).

It is also found that the majority of mutations are only moderately stabilizing or destabilizing (with transition temperatures in the range 20-60°C). In this regard, one needs to take into account that a single substitution in a peptide sequence corresponds to a triple mutation in the trimeric coiled coil structure.

It appears that e and g-mutations have a somewhat higher impact on thermal stability than b-c-substitutions. This may be partly due to the fact that e-g-positions are more buried than b-c-positions (causing a greater desolvation penalty, especially for small, polar amino acids such as serine, asparagine and aspartic acid) and partly due to the breaking of glutamine-glutamine interactions in the Q2a reference structure.

All proline mutations are disruptive, as expected for this amino acid type in alpha-helices.

The double mutations have a largely, though not completely, additive effect. For example, the two double mutants comprising the strongly destabilizing 2eD substitution (2egDR and 2egDD) are also the two least stable double mutants, but 2egDR has a higher transition temperature than 2eD despite the moderately unfavorable effect of 2gR. Possibly, pairwise electrostatic (charge-charge, ionic, Coulombic) interactions play some additional role in the double e,g-mutants, as can also be inferred from the other double mutants 2egRD, 2egRE, 2egER. However, such pairwise ionic interactions between e- and g-positions are found to be very small, if not negligible, within the context of the trimeric coiled coil scaffold. The latter is also supported by the double mutants 2egEE and 2egRR (having pairs of negative and positive charges, respectively) which do not show specific destabilizing effects that could be expected on grounds of charge-charge repulsion between e- and g-positions. In any case, none of the double mutants having side chains of opposite charge at e- and g-positions was found to require, or benefit from, ionic e,g-interactions; on the contrary, all such mutants were found to be (somewhat) less stable than the reference scaffold having glutamine at each e- and g-position. Based on these results, the present inventors conclude that for trimeric coiled coils, in contrast to dimeric coiled coils (Yu Adv Drug Deliv Rev 2002, 54:1113-1129), there is no experimental evidence that ionic e,g-interactions are required or desirable for the stability of the scaffold.

Consequently, the present invention relates to scaffolds, preferably lacking such interactions. More preferably, the present invention relates to trimeric scaffolds comprising heptad repeats which have glutamine at e- and g-positions, as is the case in the tested Q2a and Q2I reference scaffolds.

The inventors tested a representative number of amino acid substitutions at characteristic non-core positions in a trimeric coiled coil scaffold: 1 amino acid (lysine) at the very exposed f-position, 5 at the largely exposed b- and c-positions, and 10 at the moderately exposed e- and g-positions. As a rule, the more buried an amino acid is in a certain tertiary structure, the more interactions it makes with the remainder of the structure, and the more critical its nature (and changes therein) will be with respect to the stability of the tertiary structure. Reversely, the more exposed (solvent-oriented) an amino acid, the less critical will be its identity in the sense that mutations at this position will be better tolerated by (have a lower impact on) the tertiary structure. Since, for practical reasons, not all possible substitutions can be tested, the number of selected mutants was chosen in accordance with aforementioned rules regarding the correlation between degree of exposure and mutability.

Furthermore, since even at the least exposed non-core (e,g-) positions the majority of amino acid types are tolerated (have a low or moderate impact on stability), that a very large number of multiple substitutions and, hence, specific amino acid sequences other than the ones tested, will also yield stable trimeric, parallel, alpha-helical coiled coil structures. Therefore, preferred embodiments of the present invention relate to scaffold molecules formed by reference peptides Q2a and Q2aI, wherein one or more of the non-core amino acids are substituted into another amino acid type than present in the reference peptides.

One particular scaffold variant, namely the trimeric coiled coil having all-alanine at the non-core positions b, c, e, f and g, forms a special case, in that, it represents the essence of what is generally understood by a scaffold molecule, i.e., a molecule that acts as a carrier of chemical groups.

All natural amino acids (except proline) have the same backbone (main chain) atoms, but differ in the composition of their side chains.

Apart from glycine, all natural amino acids have at least one carbon atom (beta carbon, C-beta, CB) to which the remainder of the side-chain atoms are attached. Alanine only has a (hydrogen-saturated) beta carbon atom. Other amino acids (except glycine) are thus formally alanines wherein particular chemical groups are attached to the beta carbon atom. Thus, alanine represents the 'chemically pure' instance of a carrier amino acid, and proteins displaying alanines at their surface form 'chemically pure' instances of scaffold molecules onto which other side-chain types can be attached.

Accordingly, a most preferred (because chemically pure) embodiment of the present invention relates to a trimeric coiled coil scaffold wherein all non-core amino acids are alanines.

Because of the intrinsically high mutability of non-core amino acids, as demonstrated in the present example, other preferred embodiments of the present invention relate to variants of the latter scaffold wherein one or more of the non-core amino acids are substituted into another amino acid type than alanine.

Example 6. Single-chain coiled coil scaffold constructs.

To examine whether single-chain coiled coil scaffolds could be derived from peptidic coiled coils by way of connecting termini of individual heptad repeat sequences (HRS) using structurally flexible linker fragments, three constructs with different linker lengths were designed, produced and tested.

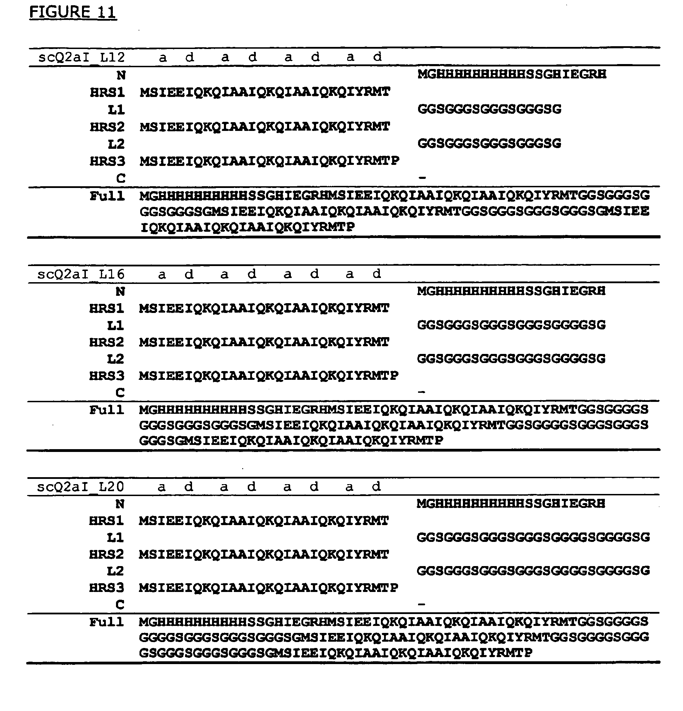

Concretely, the single-chain coiled coil scaffold molecules with the amino acid sequences listed in Figure 11 were constructed.

These scaffolds were derived from the peptidic trimeric coiled coil scaffold of Example 4 (Q2aI).

Gly/Ser-rich linkers of different lengths (12 to 20 amino acids) were tested. The constructs with linker lengths of 12, 16 and 20 amino acid residues are herein denoted as "scQ2aI_L12", "scQ2aI_L16" and "scQ2aI_L20", respectively.

In HRS1 and HRS2, the C-terminal amino acid proline was omitted from the reference Q2aI sequence.

For the design of the linkers, cassettes of glycine-glycine-serine-glycine (GGSG) were chosen.

At the N-terminus of each construct, a 10-His tag (HHHHHHHHHH) followed by a 'factor Xa' cleavage site (IEGRH) was attached. The C-termini were not modified.

Nucleotide sequences were optimized to match the codon usage for expression in *E. coli*. The genes were provided in the pCR 4 TOPO plasmid and appended with a 3'-NdeI and a 5'-XhoI restriction site for subsequent subcloning in the pET16b vector (Novagen). The tree scQ2aI_Lx/pET16b constructs were transformed into the *E. coli* BL21 (DE3)/pLysE strain and small-scale expression tests were performed.

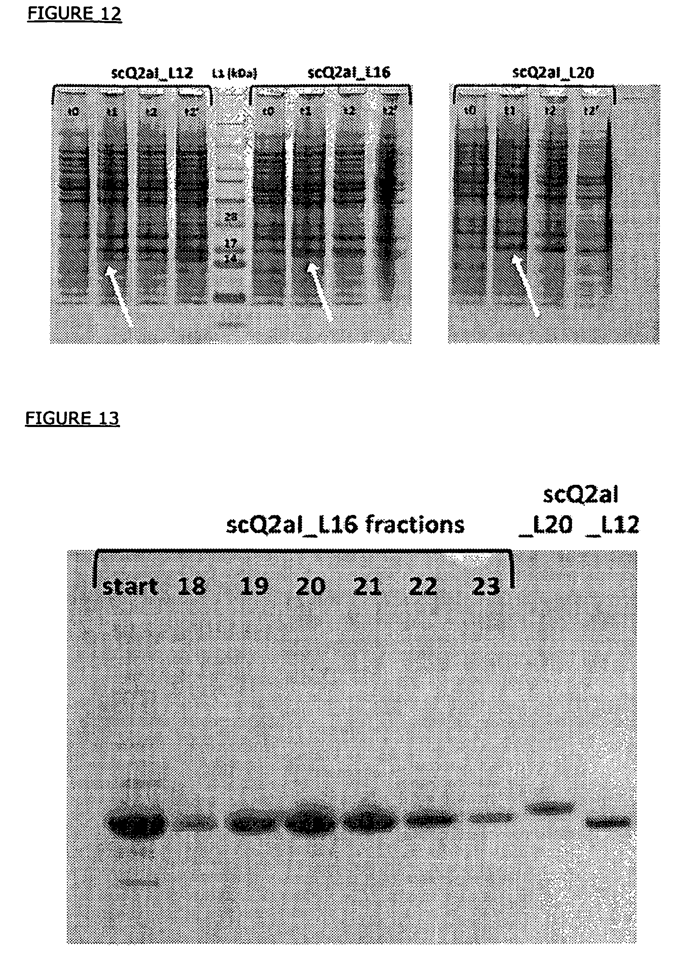

Briefly, 25 ml of medium containing the appropriate antibiotics (LB medium + 50microg/ml ampicillin + 25microg/ml chloramphenicol) was inoculated with an O/N culture (dilution 1/150x) and cells were grown at 37°C till OD600 reached about 0.65. Expression of the target proteins was then induced by the addition of 0.4 mM of IPTG and cells were further grown at either 37°C or 30°C. Culture aliquots were taken after 3.5h (t1, 37°C) and 5.5h (t2, 37°C & t2', 30°C) and analyzed on SDS-PAGE gels (10% acryl, Coomassie staining), together with a before-induction (t0) sample (Figure 12).

For all constructs, upon induction, a band (white arrows) appeared at about the expected MW of ~15 kDa.

To isolate protein from the soluble fraction, about 1.3 liter of culture was induced for 5.5h at 30°C. Cells were harvested, resuspended in a 50mM Tris, 150mM NaCl, pH 7.8 buffer and then disrupted by passing through a cell cracker. The soluble fraction was recovered by centrifugation and loaded on a 5 ml column charged with Ni2+ for IMAC-based isolation of the target protein. The column was washed with 10 column volumes of buffer containing 20mM of imidazole and a gradient of 20 to 600 mM of imidazole was used for the elution step. Protein containing fractions were pooled and concentrated from ~15 to ~6 ml (Vivaspin MWCO 5kDa, 2800 rpm). The protein was further purified on a preparative gel filtration column (Superdex 75 16/90; 50 mM Tris, 150 mM NaCl, pH 7.8 as running buffer; two runs; ~3 ml loaded/run). The proteins eluted at around 130 ml; relevant fractions were pooled and concentrated to a final volume of ~10 ml (Vivaspin MWCO 5kDa, 2800 rpm).

Figure 13 shows the relevant fractions (5 ml each) for scQ2al_L16 on SDS-PAGE, together with the previously isolated scQ2al_L20 and scQ2al_L12 proteins. Calculated soluble expression levels were ~15 mg, ~11 mg and ~13.5 mg per liter bacterial culture, for the -L12, -L16 and -L20 constructs, respectively.

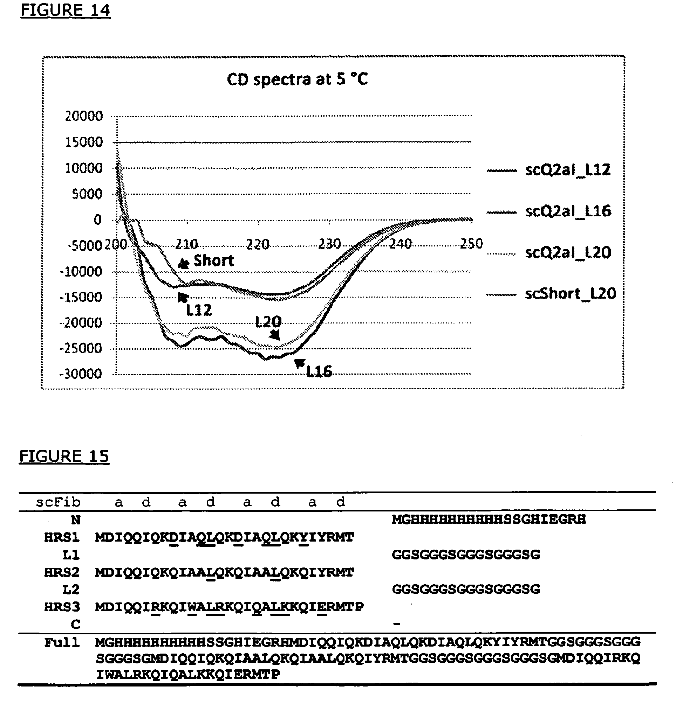

CD spectra of these constructs were recorded at 5 °C and converted to mean-residue ellipticity (Figure 14). For comparison, a variant of scQ2al_L20 named "scShort_L20" comprising a deletion of the second heptad unit and a L20 linker was also designed, produced and tested (i.e., with HRS amino acid sequences MSIEEIQKQIAAIQKQIYRMT).

It is concluded from the CD spectra in Figure 14 that all constructs display a high fraction of alpha-helical content, in agreement with the expectations.

The highest helical content is observed for scQ2al_L16, followed by scQ2al_L20, which is logical in view of its slightly larger fraction of flexible linker vs. alpha-helical coiled coil. However, the scQ2al_L12 construct shows a markedly reduced ellipticity, despite the shorter theoretically unstructured linker segments. The scQ2al_L12 spectrum almost coincides with the scShort_L20 spectrum which does seem to contain an alpha-helical content as expected for this short coiled coil construct.

This strongly suggests that the L12 linker in scQ2al_L12 is too short to bridge the distance between distal termini in a relaxed way, in other words, part of the alpha-helices are forced by the linkers to unfold. Exactly which parts (N- or C-terminal) unfold is not known, but comparison of the scQ2al_L12 and scShort_L20 spectra suggests that about 1 heptad unit is not alpha-helical. The latter result is not really surprising, since the scQ2al_L12 construct was actually developed to test the rule 'number of residues-in-linker must be at least half the number of residues-in-coiled-coil'.

Indeed, the 12-residue Gly/Ser-linker comprises less than 28/2 = 14 residues that are theoretically required (ignoring even constraints related to reversal of chain direction, i.e., turn formation at the coiled coil termini).

It is concluded from these experiments that single-chain derivatives of trimeric alpha-helical coiled coil scaffolds can be

    (i) designed,
    (ii) expressed in *E.Coli,*
    (iii) isolated in high yields from the soluble fraction,
    (iv) relatively easily purified, stored, and resolubilized, and
    (v) that these constructs display the expected alpha-helical content.

In addition, the present inventors have also performed static light scattering, analytical ultracentrifugation, thermal unfolding, long-term stability and protease resistance experiments on a variety of constructs with specific mutations and varying in length. While these results are not presented here, the inventors disclose that single-chain trimeric coiled coil scaffolds are generally robust, highly thermostable with reversible unfolding/folding transitions exceeding 100°C, relatively insensitive to (multiple) amino acid substitutions at surface-exposed positions, and can be made relatively insensitive (with degradation half-times beyond 1 h) to proteases such as pepsin and trypsin.

Example 7. Single-chain scaffold in complex with peptide.

To examine the potential of the single-chain scaffold of the present invention with respect to forming complexes with peptidic ligands in the groove formed in between HRS1 and HRS3 in the folded structure, we applied the said workable method (steps 1 to 9) disclosed in the description of the present invention.

The reference PDB template structure on which the design was based was 1OX3 ('Crystal Structure of Mini-Fibritin'), which was retrieved from the Protein Data Bank (step 1 of said workable method).

The reference single-chain scaffold (step 2) was the scQ2al_L16 construct of Example 7.

The scaffold structure was superimposed onto the trimeric coiled coil region in the 1OX3 PDB structure in different registers (step 3).

It was noticed by the present inventors that the trimeric coiled coil region harbors a peptidic, extended fragment in the groove in between each pair of adjacent alpha-helices.

It was further noticed that the said peptidic fragment is side-chain anchored into the groove, by way of amino acid residues isoleucine 3 (I3), leucine 5 (L5) and leucine 8 (L8) being buried into shallow pockets formed by coiled coil b-, c-, e- and g-positions.

Pocket-forming residues were identified (step 4) and grafted (step 5) onto the reference coiled coil scaffold of step 1.

The Brugel modeling software package was used to further optimize additional interactions.

Residues were grafted onto one side of the reference scaffold only (i.e., onto alpha-helices HRS1 and HRS3).

Since the reference scaffold was found to be of a proper length, and since it already comprised flexible linker fragments, steps 6 and 7 could be skipped.

Optimized helix-capping residues were selected (step 8) at the N-terminus by substituting the N-terminal sequences MDIQQ for the original MSIEE sequences (on all HRS).

The final amino acid sequence for the full construct (step 9) is shown in Figure 15 (grafted residues are underlined).

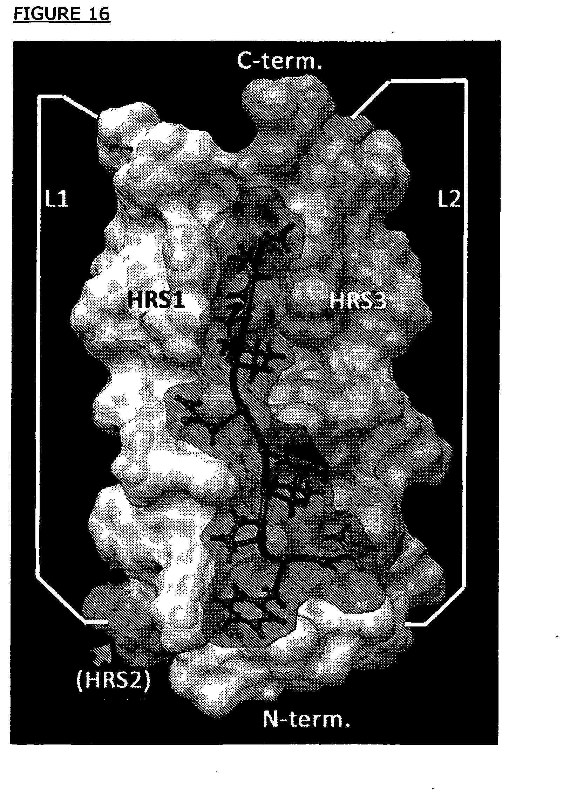

The 3-D model of the final scaffold structure, named "scFib" in complex with the 1OX3-derived peptide sequence SITLNTLPYT is shown in Figure 16.

The flexible linkers are schematically drawn as lines, because no atomic coordinates are available.

The coiled coil domain is shown in surface representation (HRS1 and HRS3 are in different shades, HRS2 is located grossly behind them), the peptide is shown in ball-and-stick representation (black) and occupies a shallow groove in between HRS1 and HRS3.

## Claims

1. A single-chain protein scaffold consisting of a full amino acid sequence of the formula HRS1-L1-HRS2-L2-HRS3, wherein HRS1, L1, HRS2, L2 and HRS3 represent amino acid sequence fragments that are covalently and consecutively interconnected in the order of the said formula, wherein

a) these fragments HRS1, HRS2 and HRS3 are heptad repeat sequences, and

b) the two fragments L1 and L2 are structurally flexible linker sequences which have an amino acid composition comprising at least 70 % of the amino acids selected from the group glycine, serine, threonine, alanine, proline, histidine, asparagine, aspartic acid, glutamine, glutamic acid, lysine, arginine, and

c) said full amino acid sequence spontaneously and stably folds in solution by way of the HRS1, HRS2 and HRS3 fragments that constitute a triple-stranded, parallel, alpha-helical coiled coil structure, and

d) each of the linkers L1 and L2 bridges between two distal ends of said coiled coil structure, and

e) the amino acid residues near the termini of each of the linkers L1 and L2 promote formation of a turn in between the connected alpha-helix and the remainder of the linker, and

f) turn-forming amino acids are selected from the group consisting of proline, glycine, asparagine and aspartic acid, and

g) such turn is made at either end of said linkers.

2. The scaffold of the claim 1, wherein the heptad repeat sequences comprise at least two consecutive 7-residue heptad repeat units of formula abcdefg.

3. The scaffold according to the claim 2, wherein at least 50%, 70%, 90% of the heptad a-positions of the 7 residues repeat units are occupied by isoleucines.

4. The scaffold of any of the claim 2 or 3, wherein all heptad a-positions of the 7 residues repeat units are occupied by isoleucines.

5. The scaffold of any of the claim 2 or 3, wherein one or more of the heptad a-positions of the 7 residues repeats units are occupied by amino acids selected from the group consisting of valine, leucine, methionine, phenylalanine, tyrosine, tryptophan, histidine, glutamine, threonine, serine, alanine, glycine.

6. The scaffold of any of the claim 2 or 3, wherein one or more of the heptad a-positions are occupied by amino acids selected from the group consisting of valine, leucine, methionine.

7. The scaffold of any of the claims 1 to 6, wherein the flexible linker sequences have an amino acid composition comprising only glycine and/or serine.

8. The scaffold of any of the claims 1 to 7, wherein the number of amino acid residues of each of the linker sequences amounts at least half of the number of amino acid residues within the coiled-coil forming region of the two heptad repeat sequences that are linked by the respective linker sequences.

9. The scaffold of any of the claims 1 to 8, wherein amino acid residues near the termini of each linker sequence are selected to stabilize helical ends of the coiled coil region.

10. The scaffold of any of the claims 1 to 9, wherein amino acid residues at conventional heptad e- and g-positions are selected to promote the stability of the said scaffold.

11. The scaffold of any of the claims 1 to 10, which remains thermodynamically stable under physical conditions that differ by at least 2 pH units (preferably 4 pH units), 20°C (preferably 40°C) and/or a factor 2 (preferably a factor 4) in ionic strength from physiological conditions being a pH of 7, a temperature of 37°C and an ionic strength of 0.15 molar.

12. The scaffold of any of the claims 1 to 11, wherein amino acid residues at conventional heptad b-, c- and f-positions are selected to promote the solubility of the said scaffold.

13. The scaffold of any of the claims 1 to 12, which remains soluble in aqueous solutions up to a concentration of 1 millimolar (preferably 1 micromolar).

14. The scaffold of any of the claims 1 to 13, wherein amino acid residues at conventional heptad g-positions in the heptad repeat sequence HRS1 and at conventional heptad e-positions in the heptad repeat sequence HRS3 form a structural indentation, or groove, into the surface of the said scaffold.

15. The scaffold of any of the claims 1 to 14, wherein conventional heptad g-positions in the heptad repeat sequence HRS1 and conventional heptad e-positions in the heptad repeat sequence HRS3 are occupied by amino acid residues of which the backbone moieties carry side chains that participate in the interface of a molecular complex between the said scaffold and a target molecule.

16. The scaffold of any of the claims 1 to 15, wherein conventional heptad g-positions in the heptad repeat sequence HRS1 and conventional heptad e-positions in the heptad repeat sequence HRS3 are occupied by amino acid residues that are selected to optimize the binding of the said scaffold to a target molecule.

17. The scaffold of any of the claims 1 to 16, wherein amino acid residues participating in the interface of a molecular complex between the said scaffold and a target molecule are optimized to such extent that the said molecular complex has a dissociation constant Kd lower than 1000 micromolar, preferably lower than 100 micromolar, more preferably lower than 10 micromolar or most preferably lower than 1 micromolar.

**Patentansprüche**

1. Einzelkettiges Proteingerüst bestehend aus einer vollständigen Aminosäuresequenz der Formel HRS1-L1-HRS2-L2-HRS3, wobei HRS1, L1, HRS2, L2 und HRS3 Aminosäuresequenzfragmente darstellen, die kovalent und durchgängig in der Reihenfolge dieser Formel verbunden sind, wobei

   a) die Fragmente HRS1, HRS2 und HRS3 Heptadenwiederholungssequenzen sind, und
   b) die zwei Fragmente L1 und L2 strukturell flexible Linkersequenzen sind, die eine Aminosäurezusammensetzung umfassend mindestens 70% der Aminosäuren ausgewählt aus der Gruppe Glycin, Serin, Threonin, Alanin, Prolin, Histidin, Asparagin, Asparaginsäure, Glutamin, Glutaminsäure, Lysin und Arginin aufweisen, und
   c) sich die vollständige Aminosäuresequenz spontan und stabil in Lösung über die HRS1-, HRS2- und HRS3-Fragmente, die eine dreisträngige parallele alpha-helikale Coiled-Coil-Struktur bilden, faltet und
   d) jeder der Linker L1 und L2 zwischen zwei distalen Enden der Coiled-Coil-Struktur eine Brücke bildet und
   e) die Aminosäurereste in der Nähe der Enden jedes der Linker L1 und L2 die Bildung einer Biegung zwischen der verbundenen alpha-Helix und dem verbleibenden Linker fördern, und
   f) biegungsförderliche Aminosäuren aus der Gruppe bestehend aus Prolin, Glycin, Asparagin und Asparaginsäure ausgewählt werden, und
   g) solch eine Biegung an einem der beiden Enden der Linker gebildet wird.

2. Gerüst gemäß Anspruch 1, wobei die Heptadenwiederholungssequenzen mindestens zwei aufeinanderfolgende 7-restige Heptadenwiederholungseinheiten der Formel abcdefg umfassen.

3. Gerüst gemäß Anspruch 2, wobei mindestens 50%, 70%, 90% der Heptade-a-Positionen der 7-restigen Wiederholungseinheiten durch Isoleucine eingenommen werden.

4. Gerüst gemäß einem der Ansprüche 2 oder 3, wobei alle Heptaden-a-Positionen der 7-restigen Wiederholungseinheiten durch Isoleucine eingenommen werden.

5. Gerüst gemäß einem der Ansprüche 2 oder 3, wobei eine oder mehrere der Heptaden-a-Positionen der 7-restigen Wiederholungseinheiten durch Aminosäuren ausgewählt aus der Gruppe bestehend aus Valin, Leucin, Methionin, Phenylalanin, Tyrosin, Tryptophan, Histidin, Glutamin, Threonin, Serin, Alanin und Glycin eingenommen werden.

6. Gerüst gemäß einem der Ansprüche 2 oder 3, wobei eine oder mehrere der Heptaden-a-Positionen durch Aminosäuren ausgewählt aus der Gruppe bestehend aus Valin, Leucin, Methionin eingenommen werden.

7. Gerüst gemäß einem der Ansprüche 1 bis 6, wobei die flexiblen Linkersequenzen eine Aminosäurezusammensetzung aufweisen, die nur Glycin und/oder Serin umfasst.

8. Gerüst gemäß einem der Ansprüche 1 bis 7, wobei sich die Anzahl der Aminosäurereste jeder der Linkersequenzen auf mindestens die Hälfte der Anzahl der Aminosäurereste in der Coiled-Coil-bildenden Region der zwei Heptadenwiederholungssequenzen, welche durch die entsprechenden Linkersequenzen verknüpft sind, beläuft.

9. Gerüst gemäß einem der Ansprüche 1 bis 8, wobei Aminosäuresequenzen in der Nähe der Termini jeder Linkersequenz ausgewählt werden, um helikale Enden der Coiled-Coil-Region zu stabilisieren.

10. Gerüst gemäß einem der Ansprüche 1 bis 9, wobei Aminosäurereste an konventionellen Heptaden-e- und Heptaden-g-Positionen ausgewählt werden, um die Stabilität des Gerüsts zu fördern.

11. Gerüst gemäß einem der Ansprüche 1 bis 10, welches unter physikalischen Bedingungen thermodynamisch stabil bleibt, die durch mindestens zwei pH-Einheiten (bevorzugt vier pH-Einheiten), 20°C (bevorzugt 40°C) und/oder einen Faktor 2 (bevorzugt einen Faktor 4) an Ionenstärke von physiologischen Bedingungen, welche einen pH von 7, eine Temperatur von 37°C und eine Ionenstärke von 0,15 Molar sind, abweichen.

12. Gerüst gemäß einem der Ansprüche 1 bis 11, wobei Aminosäurereste an konventionellen Heptaden-b-, -c- und -f-Positionen ausgewählt werden, um die Löslichkeit des Gerüsts zu fördern.

13. Gerüst gemäß einem der Ansprüche 1 bis 12, welches in wässrigen Lösungen bis zu einer Konzentration von 1 Millimolar (bevorzugt 1 Mikromolar) löslich bleibt.

14. Gerüst gemäß einem der Ansprüche 1 bis 13, wobei Aminosäurereste an konventionellen Heptaden-g-Positionen in der Heptadenwiederholungssequenz HRS1 und an konventionellen Heptaden-e-Positionen in der Heptadenwiederholungssequenz HRS3 eine strukturelle Einbuchtung oder Rille in die Oberfläche des Gerüsts bilden.

15. Gerüst gemäß einem der Ansprüche 1 bis 14, wobei konventionelle Heptaden-g-Positionen in der Heptadenwiederholungssequenz HRS1 und konventionelle Heptaden-e-Positionen in der Heptadenwiederholungssequenz HRS3 durch Aminosäurereste eingenommen werden, deren Rückgrateinheiten Seitenketten tragen, die an der Berührungsstelle eines molekularen Komplexes zwischen dem Gerüst und einem Zielmolekül beteiligt sind.

16. Gerüst gemäß einem der Ansprüche 1 bis 15, wobei konventionelle Heptaden-g-Positionen in der Heptadenwiederholungssequenz HRS1 und konventionelle Heptaden-e-Positionen in der Heptadenwiederholungssequenz HRS3 durch Aminosäurereste eingenommen werden, die ausgewählt werden, um die Bindung des Gerüsts an ein Zielmolekül zu optimieren.

17. Gerüst gemäß einem der Ansprüche 1 bis 16, wobei Aminosäurereste, die an der Berührungsstelle eines molekularen Komplexes zwischen dem Gerüst und einem Zielmolekül teilnehmen, dergestalt optimiert sind, dass der molekulare Komplex eine Dissoziationskonstante Kd von weniger als 1000 Mikromolar, bevorzugt weniger als 100 Mikromolar, noch bevorzugter weniger als 10 Mikromolar und am meisten bevorzugt weniger als 1 Mikromolar, aufweist.

**Revendications**

1. Échafaudage de protéine à chaîne unique constitué d'une pleine séquence d'acides aminés de la formule HRS1-L1-HRS2-L2-HRS3, dans laquelle HRS1, L1, HRS2, L2 et HRS3 représentent des fragments de séquence d'acides aminés qui sont interconnectés de façon covalente et consécutivement dans l'ordre de ladite formule, dans laquelle

   a) ces fragments HRS1, HRS2, et HRS3 sont des séquences de répétition en heptade, et
   b) les deux fragments L1 et L2 sont des séquences lieurs structurellement souples qui ont une composition en acides aminés comprenant au moins 70% des acides aminés choisis dans le groupe de la glycine, la sérine, la thréonine, l'alanine, la proline, l'histidine, l'asparagine, l'acide aspartique, la glutamine, l'acide glutamique, la lysine, l'arginine, et
   c) ladite pleine séquence d'acides aminés se replie spontanément et de façon stable dans une solution par le biais des fragments HRS1, HRS2 et HRS3 qui constituent une structure en faisceau d'hélices alpha-hélicoïdales, parallèles, triple brin, et
   d) chacun des lieurs L1 et L2 forme un pont entre deux extrémités distales de ladite structure en faisceau d'hélices, et
   e) les résidus d'acides aminés à proximité des extrémités de chacun des lieurs L1 et L2 favorisent la formation d'un coude entre l'hélice alpha connectée et le reste du lieur, et
   f) les acides aminés formant un coude sont choisis dans le groupe constitué par la proline, la glycine, l'asparagine et l'acide aspartique, et
   g) un tel coude est fait à chaque extrémité desdits lieurs.

2. Échafaudage selon la revendication 1, dans lequel les séquences de répétition en heptade comprennent au moins deux motifs de répétition en heptade de 7 résidus consécutifs de formule abcdefg.

3. Échafaudage selon la revendication 2, dans lequel au moins 50%, 70%, 90% des positions d'heptade a des motifs de répétition de 7 résidus sont occupées par des isoleucines.

4. Échafaudage selon l'une quelconque de la revendication 2 ou 3, dans lequel toutes les positions d'heptade a des motifs de répétition de 7 résidus sont occupées par des isoleucines.

5. Échafaudage selon l'une quelconque de la revendication 2 ou 3, dans lequel une ou plusieurs des positions d'heptade a des motifs de répétition de 7 résidus sont occupées par des acides aminés choisis dans le groupe constitué par la valine, la leucine, la méthionine, la phénylalanine, la tyrosine, le tryptophane, l'histidine, la glutamine, la thréonine, la sérine, l'alanine, la glycine.

6. Échafaudage selon l'une quelconque de la revendication 2 ou 3, dans lequel une ou plusieurs des positions d'heptade a sont occupées par des acides aminés choisis dans le groupe constitué par la valine, la leucine, la méthionine.

7. Échafaudage selon l'une quelconque des revendications 1 à 6, dans lequel les séquences lieur flexibles ont une composition en acides aminés comprenant seulement de la glycine et/ou de la sérine.

8. Échafaudage selon l'une quelconque des revendications 1 à 7, dans lequel le nombre de résidus d'acides aminés de chacune des séquences lieurs représente au moins la moitié du nombre de résidus d'acides aminés dans la région formant le faisceau d'hélices des deux séquences de répétition en heptade qui sont liées par les séquences lieurs respectives.

9. Échafaudage selon l'une quelconque des revendications 1 à 8, dans lequel les résidus d'acides aminés à proximité des extrémités de chaque séquence lieur sont sélectionnés pour stabiliser les extrémités hélicoïdales de la région du faisceau d'hélices.

10. Échafaudage selon l'une quelconque des revendications 1 à 9, dans lequel les résidus d'acides aminés aux positions d'heptade e et g conventionnelles sont sélectionnés pour favoriser la stabilité dudit échafaudage.

11. Échafaudage selon l'une quelconque des revendications 1 à 10, qui reste thermodynamiquement stable dans des conditions physiques qui diffèrent d'au moins 2 unités de pH (de préférence 4 unités de pH), 20°C (de préférence 40°C) et/ou un facteur 2 (de préférence un facteur 4) dans la force ionique par rapport à des conditions physiologiques qui sont un pH de 7, une température de 37°C et une force ionique de 0,15 molaire.

12. Échafaudage selon l'une quelconque des revendications 1 à 11, dans lequel les résidus d'acides aminés aux positions d'heptade b, c et f conventionnelles sont sélectionnés pour favoriser la solubilité dudit échafaudage.

13. Échafaudage selon l'une quelconque des revendications 1 à 12, qui reste soluble dans les solutions aqueuses jusqu'à une concentration de 1 millimolaire (de préférence 1 micromolaire).

14. Échafaudage selon l'une quelconque des revendications 1 à 13, dans lequel les résidus d'acides aminés aux positions d'heptade g conventionnelles dans la séquence de répétition en heptade HRS1 et aux positions d'heptade e conventionnelles dans la séquence de répétition en heptade HRS3 forment une indentation structurelle, ou une rainure, dans la surface dudit échafaudage.

15. Échafaudage selon l'une quelconque des revendications 1 à 14, dans lequel les positions d'heptade g conventionnelles dans la séquence de répétition en heptade HRS1 et les positions d'heptade e conventionnelles dans la séquence de répétition en heptade HRS3 sont occupées par des résidus d'acides aminés dont les fractions du squelette portent des chaînes latérales qui participent à l'interface d'un complexe moléculaire entre ledit échafaudage et une molécule cible.

16. Échafaudage selon l'une quelconque des revendications 1 à 15, dans lequel les positions d'heptade g conventionnelles dans la séquence de répétition en heptade HRS1 et les positions d'heptade e conventionnelles dans la séquence de répétition en heptade HRS3 sont occupées par des résidus d'acides aminés qui sont sélectionnés pour optimiser la liaison dudit échafaudage à une molécule cible.

17. Échafaudage selon l'une quelconque des revendications 1 à 16, dans lequel les résidus d'acides aminés participant à l'interface d'un complexe moléculaire entre ledit échafaudage et une molécule cible sont optimisés à tel point que ledit complexe moléculaire a une constante de dissociation Kd inférieure à 1 000 micromolaires, de préférence inférieure à 100 micromolaires, plus préférablement inférieure à 10 micromolaires ou au mieux inférieure à 1 micromolaire.

## FIGURE 1

**N**    **HR1**      **HR2**      **HR3**    **t**   **C**

| A | I | A | A | I | Q | K | Q | | I | A | A | L | Q | K | Q | | I | A | A | I | Q | K | Q | | I | A |

a1 b1 c1 d1 e1 f1 g1    a2 b2 c2 d2 e2 f2 g2    a3 b3 c3 d3 e3 f3 g3    a4

## FIGURE 2

| A | B | C | D | E | F | G |

FIGURE 3

FIGURE 4

## FIGURE 5

Transit. T (°C) =    46.7
ΔHt (kJ/mol) =    177.9
ΔCp (kJ/mol/K) =   *3.0

Offset 0 °C =    -34278
Slope 0 °C =    78.6
Offset 100 °C =   -7718
Slope 100 °C =    47.1

RMS Resid. =    71.5

* parameter kept fixed

## FIGURE 6

## FIGURE 7

## FIGURE 8

## FIGURE 9

## FIGURE 10

## FIGURE 11

| scQ2aI L12 | a    d    a    d    a    d    a    d |
|---|---|
| N | MGHHHHHHHHHHSSGHIEGRH |
| HRS1 | MSIEEIQKQIAAIQKQIAAIQKQIYRMT |
| L1 | GGSGGGSGGGSGGGSG |
| HRS2 | MSIEEIQKQIAAIQKQIAAIQKQIYRMT |
| L2 | GGSGGGSGGGSGGGSG |
| HRS3 | MSIEEIQKQIAAIQKQIAAIQKQIYRMTP |
| C | – |
| Full | MGHHHHHHHHHHSSGHIEGRHMSIEEIQKQIAAIQKQIAAIQKQIYRMTGGSGGGSG GGSGGGSGMSIEEIQKQIAAIQKQIAAIQKQIYRMTGGSGGGSGGGSGGGSGMSIEE IQKQIAAIQKQIAAIQKQIYRMTP |

| scQ2aI L16 | a    d    a    d    a    d    a    d |
|---|---|
| N | MGHHHHHHHHHHSSGHIEGRH |
| HRS1 | MSIEEIQKQIAAIQKQIAAIQKQIYRMT |
| L1 | GGSGGGSGGGSGGGSGGGGSG |
| HRS2 | MSIEEIQKQIAAIQKQIAAIQKQIYRMT |
| L2 | GGSGGGSGGGSGGGSGGGGSG |
| HRS3 | MSIEEIQKQIAAIQKQIAAIQKQIYRMTP |
| C | – |
| Full | MGHHHHHHHHHHSSGHIEGRHMSIEEIQKQIAAIQKQIAAIQKQIYRMTGGSGGGGS GGGSGGGSGGGGSGMSIEEIQKQIAAIQKQIAAIQKQIYRMTGGSGGGGSGGGSGGGS GGGSGMSIEEIQKQIAAIQKQIAAIQKQIYRMTP |

| scQ2aI L20 | a    d    a    d    a    d    a    d |
|---|---|
| N | MGHHHHHHHHHHSSGHIEGRH |
| HRS1 | MSIEEIQKQIAAIQKQIAAIQKQIYRMT |
| L1 | GGSGGGSGGGSGGGSGGGGSGGGGSG |
| HRS2 | MSIEEIQKQIAAIQKQIAAIQKQIYRMT |
| L2 | GGSGGGSGGGSGGGSGGGGSGGGGSG |
| HRS3 | MSIEEIQKQIAAIQKQIAAIQKQIYRMTP |
| C | – |
| Full | MGHHHHHHHHHHSSGHIEGRHMSIEEIQKQIAAIQKQIAAIQKQIYRMTGGSGGGGS GGGGSGGGSGGGSGGGSGMSIEEIQKQIAAIQKQIAAIQKQIYRMTGGSGGGGSGGGG GSGGGSGGGSGGGSGMSIEEIQKQIAAIQKQIAAIQKQIYRMTP |

FIGURE 12

FIGURE 13

FIGURE 14

CD spectra at 5 °C

scQ2al_L12
scQ2al_L16
scQ2al_L20
scShort_L20

FIGURE 15

| scFib | a   d   a   d   a   d   a   d | |
|---|---|---|
| N | | MGHHHHHHHHHHSSGHIEGRH |
| HRS1 | MDIQQIQKDIAQLQKDIAQLQKYIYRMT | |
| L1 | | GGSGGGSGGGSGGGSG |
| HRS2 | MDIQQIQKQIAALQKQIAALQKQIYRMT | |
| L2 | | GGSGGGSGGGSGGGSG |
| HRS3 | MDIQQIRRQIWALRKQIQALKKQIERMTP | |
| C | | - |
| Full | MGHHHHHHHHHHSSGHIEGRHMDIQQIQKDIAQLQKDIAQLQKYIYRMTGGSGGGSGGG | |
| | SGGGSGMDIQQIQKQIAALQKQIAALQKQIYRMTGGSGGGSGGGSGGGSGMDIQQIRRQ | |
| | IWALRKQIQALKKQIERMTP | |

FIGURE 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5824483 A

- US 2003002795 A

**Non-patent literature cited in the description**

- **EFIMOV VP et al.** Fibritin Encoded by Bacteriophage T4 Gene wac has a Parallel Triple-stranded .alpha.-Helical Coiled-coil Structure. *JOURNAL OF MOLECULAR BIOLOGY,* 1994, vol. 242, 470-486
- **Hosse et al.** *Protein Sci,* 2006, vol. 15, 14-27
- **Burkhard et al.** *Trends Cell Biol,* 2001, vol. 11, 82-88
- **Wolf et al.** *Protein Sci,* 1997, vol. 6, 1179-1189
- *Lupas Trends Biochem Sci,* 1996, vol. 21, 375-382
- **Naik et al.** *Biosens Bioelectron,* 2001, vol. 16, 1051-1057
- **Arndt et al.** *J Mol Biol,* 2001, vol. 312, 221-227
- **Moitra et al.** *Genes Dev,* 1998, vol. 12, 3168-3181
- **Müller et al.** *Methods Enzymol,* 2000, vol. 328, 261-282
- **Wang et al.** *Nature,* 1999, vol. 397, 417-420
- **Ryadnov et al.** *J Am Chem Soc,* 2003, vol. 125, 9388-9394
- **Mason ; Arndt.** *ChemBioChem,* 2004, vol. 5, 170-176
- **Wolf et al.** *Protein Science,* 1997, vol. 6, 1179-1189

- **Eckert ; Kim.** *Proc Natl Acad Sci USA,* 2001, vol. 98, 11187-11192
- **Yin et al.** *Nature,* 2006, vol. 439, 38-44
- **Suzuki et al.** *Protein Eng,* 1998, vol. 11, 1051-1055
- **Harbury et al.** *Science,* 1993, vol. 262, 1901-1407
- *Nature,* 1994, vol. 371, 80-83
- *Nautiyal and Alber Protein Sci,* 1999, vol. 8, 84-90
- *Yu Adv Drug Deliv Rev,* 2002, vol. 54, 1113-1129
- **Lovejoy et al.** *Science,* 1993, vol. 259, 1288-1293
- **Dragan ; Privalov.** *J mol Biol,* 2002, vol. 321, 891-908
- **Dragan et al.** *Biochemistry,* 2004, vol. 43, 14891-14900
- **Skerra.** *J Mol Recognit,* 2000, vol. 13, 167-187
- **Eckert et al.** *J Mol Biol,* 1998, vol. 284, 859-865
- **Harbury et al.** *Science,* 1993, vol. 262, 1401-1407

SEQUENCE LISTING

<110> Complix NV

<120> SINGLE-CHAIN COILED COIL SCAFFOLD

<130> ALGO 14

<160> 5

<170> PatentIn version 3.3

<210> 1
<211> 24
<212> PRT
<213> Artificial sequence

<220>
<223> Synthetic peptide (Example 1)

<400> 1

```
Ala Ile Ala Ala Ile Gln Lys Gln Ile Ala Ala Leu Gln Lys Gln Ile
1               5                   10                  15

Ala Ala Ile Gln Lys Gln Ile Ala
                20
```

<210> 2
<211> 29
<212> PRT
<213> Artificial sequence

<220>
<223> synthetic peptide (Example 2)

<400> 2

```
Met Ser Ile Glu Glu Ile Gln Lys Gln Gln Ala Ala Ile Gln Lys Gln
1               5                   10                  15

Ile Ala Ala Ile Gln Lys Gln Ile Tyr Arg Met Thr Pro
                20                  25
```

<210> 3
<211> 29
<212> PRT
<213> Artificial sequence

<220>
<223> Synthetic peptide (Example 3)

<400> 3

```
Met Ser Ile Glu Glu Ile Gln Lys Gln Ile Ala Ala Ile Gln Lys Gln
1               5               10              15

Ile Ala Ala Ile Gln Lys Gln Ile Tyr Arg Met Thr Pro
            20              25
```

<210> 4
<211> 21
<212> PRT
<213> Artificial sequence

<220>
<223> synthetic primer (Example 6; scShort_L20)

<400> 4

```
Met Ser Ile Glu Glu Ile Gln Lys Gln Ile Ala Ala Ile Gln Lys Gln
1               5               10              15

Ile Tyr Arg Met Thr
            20
```

<210> 5
<211> 10
<212> PRT
<213> Artificial sequence

<220>
<223> Synthetic paptide (Example 7; scFib)

<400> 5

```
Ser Ile Thr Leu Asn Thr Leu Pro Tyr Thr
1               5               10
```